# EUROPEAN PATENT SPECIFICATION

(11) **EP 1 869 191 B1**
(45) Date of publication and mention of the grant of the patent: **26.12.2012**
(21) Application number: 06717191.8
(22) Date of filing: 22.03.2006
(51) Int. Cl.: C07K 14/47, C12N 15/86

(54) **NOVEL NEURAL CELL SPECIFIC PROMOTER AND BACULOVIRUS AND METHOD FOR GENE DELIVERY**
NEUER NEURALZELLEN-SPEZIFISCHER PROMOTOR SOWIE BACULOVIRUS UND VERFAHREN ZUR GENZUFÜHRUNG
NOUVEAU PROMOTEUR SPÉCIFIQUE NEURAL CELLULAIRE ET BACULOVIRUS, ET PROCÉDÉ D'APPORT DE GÈNES

(30) Priority: 22.03.2005 US 663873 P
(43) Date of publication of application: 26.12.2007
(73) Proprietor: Agency for Science, Technology and Research, Singapore 138668 (SG)
(72) Inventor: Wang, Shu, Singapore 138669 (SG); Wang, Chao Yang, Singapore 138669 (SG)
(74) Representative: Kremer, Simon Mark
(86) International application number: PCT/SG2006/000067
(87) International publication number: WO 2006/101457

(56) References cited:
- EP-A- 1 336 652
- WO-A-96/09074
- WO-A-98/45462
- WO-A-03/093295
- WO-A-2004/067751
- WO-A-2004/087926
- WO-A2-01/89583
- US-A1- 2003 148 506
- US-A1- 2004 197 313
- US-B1- 6 503 888
- FITZSIMONS H.L. ET AL.: 'Promoters and regulatory elements that improve adeno-associated virus transgene expression in the brain' METHODS vol. 28, no. 2, 2002, pages 227 - 236, XP008121222
- CHEN J. ET AL.: 'A Glial-specific, Repressible, Adenovirus Vector for Brain Tumor Gene Therapy' CANCER RESEARCH vol. 58, no. 16, 1998, pages 3504 - 3507, XP008121223
- LI Y. ET AL.: 'Neuronal gene transfer by baculovirus-derived vectors accommodating a neurone-specific promoter' EXPERIMENTAL PHYSIOLOGY vol. 90, no. 1, 12 November 2004, pages 39 - 44, XP008121224
- LIU B.H. ET AL.: 'CMV enhancer/human PDGF-beta promoter for neuron-specific transgene expression' GENE THERAPY vol. 11, no. 1, 2004, pages 52 - 60, XP009108384
- WANG C.Y. ET AL.: 'Improved neuronal transgene expression from an AAV-2 vector with a hybrid CMV enhancer/PDGF-beta promoter' THE JOURNAL OF GENE MEDICINE vol. 7, no. 7, 09 March 2005, pages 945 - 955, XP008121243

### SEQUENCE LISTING

<110> Wang, Shu
   Wang, chao Yang
<120> NOVEL NEURAL SPECIFIC PROMOTER AND BACULOVIRUS AND METHOD FOR GENE DELIVERY
<130> 93231-97
<160> 10
<170> PatentIn version 3.3
<210> 1
   <211> 1675
   <212> DNA
   <213> human
<220>
   <221> misc_feature
   <223> GFAP promoter region from human
<400> 1
   gtctgcaagc agacctggca gcattgggct ggccgccccc cagggcctcc tcttcatgcc      60
   cagtgaatga ctcaccttgg cacagacaca atgttcgggg tgggcacagt gcctgcttcc     120
   cgccgcaccc cagcccccct caaatgcctt ccgagaagcc cattgagtag ggggcttgca     180
   ttgcacccca gcctgacagc ctggcatctt gggataaaag cagcacagcc ccctaggggc     240
   tgcccttgct gtgtggcgcc accggcggtg gagaacaagg ctctattcag cctgtgccca     300
   ggaaagggga tcaggggatg cccaggcatg gacagtgggt ggcagggggg gagaggaggg     360
   ctgtctgctt cccagaagtc caaggacaca aatgggtgag gggactgggc agggttctga     420
   ccctgtggga ccagagtgga gggcgtagat ggacctgaag tctccaggga caacagggcc     480
   caggtctcag gctcctagtt gggcccagtg gctccagcgt ttccaaaccc atccatcccc     540
   agaggttctt cccatctctc caggctgatg tgtgggaact cgaggaaata aatctccagt     600
   gggagacgga ggggtggcca gggaaacggg gcgctgcagg aataaagacg agccagcaca     660
   gccagctcat gcgtaacggc tttgtggagc tgtcaaggcc tggtctctgg gagagaggca     720
   cagggaggcc agacaaggaa ggggtgacct ggagggacag atccaggggc taaagtcctg     780
   ataaggcaag agagtgccgg ccccctcttg ccctatcagg acctccactg ccacatagag     840
   gccatgattg acccttagac aaagggctgg tgtccaatcc cagcccccag ccccagaact     900
   ccagggaatg aatgggcaga gagcaggaat gtgggacatc tgtgttcaag ggaaggactc     960
   caggagtctg ctgggaatga ggcctagtag gaaatgaggt ggcccttgag ggtacagaac    1020
   aggttcattc ttcgccaaat tcccagcacc ttgcaggcac ttacagctga gtgagataat    1080
   gcctgggtta tgaaatcaaa aagttggaaa gcaggtcaga ggtcatctgg tacagccctt    1140
   ccttcccttt tttttttttt tttttttttg tgagacaagg tctctctctg ttgcccaggc 1200
   tggagtggcg caaacacagc tcactgcagc ctcaacctac tgggctcaag caatcctcca 1260
   gcctcagcct cccaaagtgc tgggattaca agcatgagcc accccactca gccctttcct 1320
   tcctttttaa ttgatgcata ataattgtaa gtattcatca tggtccaacc aaccctttct 1380
   tgacccacct tcctagagag agggtcctct tgattcagcg gtcagggccc cagacccatg 1440
   gtctggctcc aggtaccacc tgcctcatgc aggagttggc gtgcccagga agctctgcct 1500
   ctgggcacag tgacctcagt ggggtgaggg gagctctccc catagctggg ctgcggccca 1560
   accccacccc ctcaggctat gccagggggt gttgccaggg gcacccgggc atcgccagtc 1620
   tagcccactc cttcataaag ccctcgcatc ccaggagcga gcagagccag agcat 1675
<210> 2
   <211> 1604
   <212> DNA
   <213> rat
<220>
   <221> misc_feature
   <223> GFAP promoter region from rat
<400> 2
<210> 3
   <211> 387
   <212> DNA
   <213> cytomegalovirus
<220>
   <221> misc_feature
   <223> CMV enhancer (-568 to -187 relative to the TATA box of the CMV immediate-early promoter)
<400> 3
   cctgggtcga cattgattat tgactagtta ttaatagtaa tcaatacggg gtcattagtt 60
   catagcccat atatggagtt ccgcgttaca taacttacgg taaatggccc gcctggctga 120
   ccgcccaacg acccccgccc attgacgtca ataatgacgt atgttcccat agtaacgcca 180
   atagggactt tccattgacg tcaatgggtg gactatttac ggtaaactgc ccacttggca 240
   gtacatcaag tgtatcatat gccaagtacg ccccctattg acgtcaatga cggtaaatgg 300
   ccccctggca ttatgcccag tacatgacct tatgggactt tctacttggc agtacatcta 360
   cgtattagtc atcgctatta ccatggt 387
<210> 4
   <211> 146
   <212> DNA
   <213> adeno-associated virus
<220>
   <221> misc_feature
   <223> inverted Terminal Repeat sequence of Adeno-Associated virus
<400> 4
   ttggccactc cctctctgcg cgctcgctcg ctcactgagg ccgggcgacc aaaggtcgcc 60
   cgacgcccgg gctttgcccg ggcggcctca gtgagcgagc gagcgcgcag agagggagtg 120
   gccaactcca tcactagggg ttcctg 146
<210> 5
   <211> 2166
   <212> DNA
   <213> artificial
<220>
   <223> upstream construct of hybrid promoter and upstream itr sequence
<400> 5
   cctgcaggca gctgcgcgct cgctcgctca ctgaggccgc ccgggcaaag cccgggcgtc 60
   gggcgacctt tggtcgcccg gcctcagtga gcgagcgagc gcgcagagag ggagtggcca 120
   actccatcac taggggttcc tgcggccgcg gtacccctgg gtcgacattg attattgact 180
   agttattaat agtaatcaat acggggtcat tagttcatag cccatatatg gagttccgcg 240
   ttacataact tacggtaaat ggcccgcctg gctgaccgcc caacgacccc cgcccattga 300
   cgtcaataat gacgtatgtt cccatagtaa cgccaatagg gactttccat tgacgtcaat 360
   gggtggacta tttacggtaa actgcccact tggcagtaca tcaagtgtat catatgccaa 420
   gtacgccccc tattgacgtc aatgacggta aatggccccc tggcattatg cccagtacat 480
   gaccttatgg gactttctac ttggcagtac atctacgtat tagtcatcgc tattaccatg 540
   gtgagctctt acgcgtgcta gccctgcagg gcccactagt ctgtaagctg gaagtctggc 600
   agtgctgagc tggccaaccc cctcaggacc tcctccttgt gcccactgaa tgactcacct 660
   tggcatagac ataatggtca ggggcgggca cacagcctga ttcccgctgc actccaggcc 720
   cccttcaatg ctttccgaga agtccattga gctgggagct tgtactgcac caagggctga 780
   catcctggca gccagggatg aaagcagccc atggggctac ccttgccgta tgcctcactg 840
   gcggcagaga acaaggctct attcagcaaa tgccctggag tagacaccag aagtccaagc 900
   atgggcagag gaaggcaggc gttgggggct ggaggggagc agagctgtct gttttccaga 960
   agcccaaggg tacagatggc gcctgggggg gaactgagtg gaggggatag atgggcctga 1020
   gatctcaaac atcaacagcc tcctccccac caacgatgaa ggtggaggtt ggtttcccag 1080
   acctacatat cccccagaga cctggtgtat gaaaattcaa aggaggtaag tctcctgaga 1140
   gaacgggggg ctcacaaatg aagccagctg tcttacccta tcaggaccta cgtgcattcc 1200
   ttctgtcctg ccccctaaac acacagccag aggctcaaat tgattctgga gtcacaaagg 1260
   gggcttgaaa ccccagcccc ccactcctga actccaggaa tgagaagata gtattggagg 1320
   ggttcagagg agagggctct gcacatctgt tgagaatggg ggtcccagga gagtgtaatt 1380
   taggctgatc ccggaggaag ggaataggct cttcaagatc ctagcatctc acaggcccac 1440
   agagaagttc agagttgggg cagccctggc ttacaggctc taagaactgg aggcagttta 1500
   cccaacccag ctgtgtgcat gctgtccctc tctctgtctc tgtctgtctc tctctgtctc 1560
   tgtctctctg tgtgtgtgtg tgtgtgctca cacacgtgtg tgtttatcac acaaatgttc 1620
   atgtgtgtgt acatacatgt gttgaggcca gaggtcaacc tcagacactg ttgacttggt 1680
   tgtatgagat aacatttccc cctgggacct gggatttgcc aattagtgtg acccaggaag 1740
   cctacttatt ttcattcctc agcactgcag ttacaagtat gcactgtcaa accaggcctt 1800
   tttttttttt tttttccaaa ccaggccttt tgtattcgct ctgtggctag aacttgggtc 1860
   tccatgcttg acaggcaagc gatttatgga ctaagctgtt tcctcggccc tctcttgacc 1920
   catttaccag aaatggggtt tccttgatca atggttaagc caggctggtg ttcccaggaa 1980
   acccttgact ctgggtacag tgaccttggt ggggtgagaa gagttctctc catagctggg 2040
   ctggggccca gctccacccc ctcaggctat tcaatggggt gctgccagga agtcaggggc 2100
   agatccagtc cagcccgtcc ctcaataaag gccctgacat cccaggagcc agcagaagca 2160
   gggcat 2166
<210> 6
   <211> 141
   <212> DNA
   <213> artificial
<220>
   <223> downstream itr sequence
<400> 6
   aggaacccct agtgatggag ttggccactc cctctctgcg cgctcgctcg ctcactgagg 60
   ccgggcgacc aaaggtcgcc cgacgcccgg gctttgcccg ggcggcctca gtgagcgagc 120
   gagcgcgcag ctgcctgcag g 141
<210> 7
   <211> 17
   <212> DNA
   <213> artificial
<220>
   <223> synthetic primer
<400> 7
   cgccctggtt cctggaa 17
<210> 8
   <211> 25
   <212> DNA
   <213> artificial
<220>
   <223> synthetic primer
<400> 8
   ggacatttcg aagtactcag cgtaa 25
<210> 9
   <211> 19
   <212> DNA
   <213> artificial
<220>
   <223> synthetic primer
<400> 9
   atgtccacct cgatatgtg 19
<210> 10
   <211> 1488
   <212> DNA
   <213> human
<400> 10
   ctagaggatc cacagtctcc tgagtagctg ggactacagg agcttgttac cacacccagc 60
   tccagtttat aaattcatct ccagtttata aaggaggaaa ccgaggtact gagaggttaa 120
   aaaaccttcc tgcagacact tgtccagcaa gtggccactc caggatttgg accaaggtga 180
   tgtgtcttca ggctgtgtct ctgccactgt gccacgctgc tgggtggtag gcagcagtgg 240
   gtgggtgcct gcagtggtct gtaaagacca cctgagatgt ccttcctcct ctgttccacc 300
   ctgtccaggt ccaagaagac agtctatgaa gagagagcag gtgtgactct ctcagtgtgc 360
   tcctctgtga gaagcaggct gacatcccaa agggaagggc ggataacaga gacagtgcaa 420
   gcggaggaga tgagggtgcc tcaaagccgg gaggctgggt gatgcaggag cctgcgtgtc 480
   ccgagggggg tgctgggccc agtgtgagta cgtgtgactg tgactgagac agtgtgactg 540
   ctgaaggcag ggacacagca gctccctgac tgggggcaga aggcgttaac tgtgtgaagg 600
   ctggttgtgg gtgggtgggc tctgggcctc gaacccgggg gctgagggag atagtaaaca 660
   gcagggtgac tgacgggaag atcatgttgg tagccctgcg aagatgctgc agggctgtgg 720
   gggtttgtgt gactttgcag ttcaacaaat tcaaattcag ccaacgctgg cagggcctgt 780
   tgtgccaggc aaccagctag gaggaggaga ctcggaccca gcttgcagct gaagggcgct 840
   ggctgccggg ttctgtgggt tcaccttgcg gtgtcttccc ttgctaacac tgagtcctta 900
   caatagcccc atctccaggt tgaggctaga tggaggggac agagggaagt gacttgccca 960
   aggtgaccca agctcccgag tgccagggca ggatctgaat tcaggctctc agactgcaga 1020
   gcctgagtcc ctccctgcca tgcctgtgcc agggtggaaa tgtctggtcc tggaggggag 1080
   cgtggactcc tggccttggc tctggagaca tccccctaga ccacgtgggc tcctaacctg 1140
   tccatggtca ctgtgctgag gggcgggacg gtgggtcacc cctagttctt ttttccccag   1200
   ggccagattc atggactgaa gggttgctcg gctctcagag accccctaag cgccccgccc   1260
   tggccccaag ccctccccca gctcccgcgt cccccccctc ctggcgctga ctccgggcca   1320
   gaagaggaaa ggctgtctcc acccacctct cgcactctcc cttctccttt ataaaggccg   1380
   gaacagctga aagggtggca acttctcctc ctgcagccgg gagcggcctg cctgcctccc   1440
   tgcgcacccg cagcctcccc cgctgcctcc ctagagtcga ggaactaa                 1488

## Description

### CROSS-REFERENCE TO RELATED APPLICATION

This application claims benefit and priority from U.S. provisional patent application No. 60/663,873, filed on March 22, 2005.

### FIELD OF THE INVENTION

The present invention relates generally to delivery of nucleic acids to neural cells, including neuronal and glial cells.

### BACKGROUND OF THE INVENTION

Neural cells that make up the nervous system and include neurons, cells involved in transmission of nerve impulses, and glial cells, the cells that support neurons but which are not involved in nerve impulse transmission. Gene mutations or disruptions, or cellular events that alter the normal physiology of these cells, can lead to diseases of the nervous system, or neural-related disorders or diseases.

Neurons have been implicated in many different physiological functions and are highly sensitive to cellular changes, often resulting in dysfunction of the cells. Neurons are involved in various neuronal-related diseases and disorders, including neurodegenerative disorders and cancers such as neuronal cancers, including medulloblastomas, and neuroblastomas.

Glial cells are involved in a number of disease conditions or disorders, including neurodegenerative disorders such as Alzheimer's disease and Parkinson's disease, as well as cancers such as gliomas and astrocytomas, all of which glial-related diseases and disorders are potentially treatable using a gene therapy approach.

Gliomas are the most common type of intracranial tumours, and have a tendency to invade rapidly in the brain. These tumours have recently been one of the major focuses of cancer therapy (Holland, E.C., Proc Natl Acad Sci USA (2000) 97:6242-4). Gliomas are among the most lethal of all cancers and do not respond well to current therapies. These tumours originate from glial cells, predominantly from astrocytes, and are graded from I to IV with increasing level of malignancy. Grade IV gliomas, also named glioblastoma multiforme (GBM), comprise nearly half of all gliomas and are the most frequent primary brain tumours in adults. GBM is currently almost incurable. Even treated with surgery, radiotherapy, and chemotherapy, patients with GBM usually die within a year, with only few patients surviving longer than 3 years.

Gene therapy has now been viewed as a new promising therapeutic modality for cancers, having been successfully applied using various types of viral vectors, gene expression regulation elements and putative anti-tumour genes in animal models. Gene therapy methods are being tested as either an alternative way to eliminate tumour cells directly or as an addition to traditional treatments in order to augment effectiveness of treatment. Of over 400 clinical trials of gene therapy that were carried out in the last 15 years, almost 70% were related to cancer therapy (El-Aneed, A., J Control Release. (2004) Jan 8;94(1):1-14).

A potential advantage of gene therapy is to restrict expression of a therapeutic gene of interest to diseased cells and leave neighbouring normal cells unaffected by the therapeutic gene expression. Such specific gene expression has the potential to limit side effects caused by the expression of exogenous genes in non-target cells, including systemic toxicity, inflammatory reactions and immune responses, while ensuring therapeutic efficacy in diseased cells. This approach would be extremely valuable for gene therapy in the central nervous system (CNS), including in neuronal cells or glial cells.

Specific gene expression in a selected cell type can be achieved at the level of targeted gene delivery through the use of ligand-associated delivery vectors and the binding of ligands to the cell surface receptors that are unique to target cells.

Specific gene expression can also be achieved at the level of targeted expression through the use of cell-specific promoters. The use of cell-specific promoters has the advantage that such promoters are less likely to activate intracellular defence machinery because of their authentic cellular sequences, thus being less sensitive to promoter silencing as compared to viral promoters (Dressel, U. et al., Anticancer Res (2000) 20:1017-1022). However, many cell specific mammalian promoters display a relatively weak transcriptional activity when compared to widely used strong promoters derived from viruses, for example the cytomegalovirus immediately early promoter and enhancer (CMV promoter).

WO 98/45462 describes vectors derived from recombinant Adeno-associated virus (AVV) which comprise at least one selected transgene between the sequences of the 5' and 3' inverted terminal-repeats (ITRs) from AAV, and a DNA sequence encoding one or more AAV Rep protein, or a fragment or a derivative thereof, outside of the context of the AAV ITRs.

US 2003/148506 A1 describes a method of producing an adeno-associated virus (AAV) in an insect cell comprising (i) providing at least one insect cell-compatible vector comprising a first nucleotide sequence comprising at least one AAV ITR nucleotide sequence, a second nucleotide sequence containing an open reading frame encoding AAV VPI, VP2, and VP3 capsid proteins, a third nucleotide sequence comprising a Rep52 or a Rep40 coding sequence, and a fourth nucleotide sequence comprising a Rep78 or a Rep68 coding sequence, (ii) introducing the at least one insect cell-compatible vector into an insect cell, and (iii) maintaining the insect cell under conditions such that AAV is produced.

WO 96/09074 A describes a method of expressing an exogenous gene in a mammalian cell, involving infecting the cell with a non-mammalian virus such as a baculovirus, whose genome carries an exogenous gene, and growing the cell under conditions such that the gene is expressed.

WO 2004/087926 A describes a chimeric promoter construct useful for neuronal cell specific gene expression. Also described is a recombinant nucleic acid molecule that comprises a neuronal cell specific promoter such as platelet-derived growth factor ß-chain promoter, operably linked to a heterologous enhancer which enhances the transcriptional activity of the promoter, such as cytomegalovirus immediate early gene enhancer.

US-B1-6 503 888 describes a method of delivering exogenous DNA to a target cell of the mammalian central nervous system using an adeno-associated virus (AVV) derived vector. Also described are the AAV-derived vectors containing exogenous DNA which encodes a protein or proteins which prevent or treat nervous system disease, and a method of prevent or treating such disease.

FITZSIMONS H.L. ET AL., METHODS, vol. 28, no. 2, 2002, pages 227 - 236, describes different promoters and regulatory elements that have been used with rAAV as a reference toward achieving a high level of rAAV-mediated transgene expression in the CNS.

EP-A-1 336 652 describes a cell line for producing a virus vector without resort to any troublesome operations and provide a process for producing a virus vector having a high titer with the use of the cell line. Namely, cells to be used in producing a virus vector having an antisense gene been transferred there into are provided, wherein the gene expresses an antisense RNA against the whole or partial sequence of a sense RNA expressed by a gene encoding a cytotoxic polypeptide.

WO 03/093295 A describes delivery vectors for transferring a nucleic acid sequence to a cell in vitro, ex vivo or in vivo. The delivery vector comprises a segment encoding a secretory signal peptide.

WO 2004/067751 A describes the use of regulatory sequences for mediating specific, early transient expression neuronal determined cells. Furthermore, the use of recombinant nucleic acid molecules comprising said defined regulatory sequences for mediating specific, early transient expression in proliverative neuronal determined cells as well as for the generation of non-human transgenic organisms and/or ghost cells is described. In addition, the document describes transgenic non-human animals and/or host cells comprising said regulatory sequences and/or recombinant nucleic acid molecules. The preparation of such vectors, host cells and transgenic nonhuman animals as well as methods for the detection and/or isolation of neuronal determined cells is described.

WANG C.Y. ET AL., THE JOURNAL OF GENE MEDICINE, vol. 7, no. 7, 9 March 2005, pages 945-955, describes the development of AAV-2 vectors that also facilitate a high level of neuronal expression by enhancing the strength of a neuron-specific promoter, the human platelet-derived growth factor β-chain (PDGF) promoter.

Many approaches have been developed to improve the strength of a cellular promoter. However, not all cellular promoters are sensitive to this enhancement method. In one of the previous efforts that tested 19 different gene regulatory elements produced by combination of a muscle-specific promoter with the CMV enhancer/promoter, only one offered significant increase in transgene expression over a control (Barnhart et al., Hum Gene Ther (1998) 9:2545-2553).

### SUMMARY OF THE INVENTION

Cell type-specific promoters are of great interest to people working in therapeutic fields because of their potential in driving selective gene expression in target cells. In an effort to provide approaches for treating certain neural-related disorders, including neuronal-related disorders and glial-related disorders, the inventors have developed a novel neural-specific hybrid promoter to drive expression of a therapeutic agent in neural cells and in cells derived from neural-cells. The novel neural-specific promoter can be used in the context of an expression cassette, which can be included in any nucleic acid vector, but is particularly useful when included in a baculovirus, since baculovirus can enter mammalian cells but does not express viral genes or replicate in mammalian cells.

Thus, the present invention provides a baculovirus vector comprising an expression cassette, the expression cassette comprising, (i) a glial fibrillary acidic protein (GFAP) promoter operably linked to a heterologous enhancer, (ii) one or both of a coding sequence operably linked to the GFAP promoter and a cloning site for inserting a coding sequence operably linked to the GFAP promoter, and (iii) a pair of viral inverted terminal repeats from adeno-associated virus, said pair of viral inverted terminal repeats flanking at least the operably linked GFAP promoter and said one or both of the coding sequence and the cloning site;wherein the heterologous enhancer increases the glial-specific transcriptional activity of the GFAP promoter.

The pair of viral inverted terminal repeats may also flank the heterologous enhancer. The inverted terminal repeats may comprise the sequence set forth in SEQ ID NO: 4.

The GFAP promoter may comprise the sequence set forth in SEQ ID NO: 1 or SEQ ID NO: 2.

The heterologous enhancer may be a viral enhancer, for example a cytomegalovirus immediate early enhancer or SV40 enhancer. In embodiments where the enhancer is a cytomegalovirus immediate early enhancer, the enhancer may comprise the region -568 to -187 base pairs relative to the TATA box of the cytomegalovirus immediate early promoter; for example. The enhancer may have the sequence set forth in SEQ ID NO: 3. The heterologous enhancer may be operably linked upstream to the GFAP promoter.

The baculovirus vector may comprise the sequence as set forth in SEQ ID NO: 5 upstream of the coding sequence or cloning site and the sequence as set forth in SEQ ID NO: 6 downstream of the coding sequence or cloning site.

The coding sequence may encode a therapeutic product.

The therapeutic product may be a protein, a peptide, a ribozyme, a small interfering RNA, a microRNA or an antisense RNA. In embodiments where the therapeutic product is a protein or peptide, the product may be involved in the treatment of a neural-related disorder. The therapeutic product may be a neurotrophic factor, a growth factor, an anti-apoptotic protein, a cytotoxin, an apoptotic protein, a tumour suppressor protein, an immunomodulator protein, an oncoprotein, an antibody or an anti-angiogenic protein. For example, the therapeutic product may be p53, a p53 pathway protein or a DT-A bacterial protein.

The neural-related disorder may be a neuronal cancer, a medulloblastoma, a neuroblastoma, a glioma, an astrocytoma, a neurodegenerative disorder, Alzheimer's disease, Parkinson's disease, epilepsy or damage as a result of alcohol exposure.

The present invention also provides an in vitro method of expressing a nucleic acid in a glial cell or in a cell derived from a glial cell, comprising transfecting in vitro a glial cell or a cell derived from a glial cell with the baculovirus vector as defined above in this summary of invention.

The present invention further provides a baculovirus vector as defined above in this summary of invention for use in a method of treating a neural-related disorder in a subject, the method comprising expressing a nucleic acid in a glial cell or in a cell derived from a glial cell, wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

Also provided is a baculovirus vector for use in a method of treating a neural-related disorder in a subject, the baculovirus vector comprising an expression cassette, the expression cassette comprising, (i) a glial fibrillary acidic protein (GFAP) promoter operably linked to a heterologous enhancer, (ii) a coding sequence operably linked to the GFAP promoter, and (iii) a pair of viral inverted terminal repeats from adeno-associated virus, said pair of viral inverted terminal repeats flanking at least the operably linked GFAP promoter and coding sequence; wherein the heterologous enhancer increases the glial-specific transcriptional activity of the GFAP promoter; and wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

In one aspect the invention provides for use of a baculovirus vector for preparation of a medicament for treating a neural-related disorder in a subject, the baculovirus vector comprising an expression cassette, the expression cassette comprising, (i) a glial fibrillary acidic protein (GFAP) promoter operably linked to a heterologous enhancer, (ii) a coding sequence operably linked to the GFAP-specific promoter, and (iii) a pair of viral inverted terminal repeats from adeno-associated virus, said pair of viral inverted terminal repeats flanking at least the operably linked GFAP promoter and coding sequence; wherein the heterologous enhancer increases the glial-specific transcriptional activity of the GFAP promoter; and wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

The expression cassette of the above described vector for use and uses per se may be the expression cassette as defined in this summary of invention. The neural-related disorder may be a neuronal cancer, a medulloblastoma, a neuroblastoma, a glioma, an astrocytoma, a neurodegenerative disorder, Alzheimer's disease, Parkinson's disease, epilepsy or damage as a result of alcohol exposure.

The invention provides a transgenic cell comprising the baculovirus vector as defined above in this summary of invention.

Also provided is a non-human animal comprising the baculovirus vector as defined above in this summary of invention.

Further provided is a pharmaceutical composition comprising the baculovirus vector as defined above in this summary of invention.

Other aspects and features of the present invention will become apparent to those of ordinary skill in the art upon review of the following description of specific embodiments of the invention in conjunction with the accompanying figures.

### BRIEF DESCRIPTION OF THE DRAWINGS

**FIG. 1** is a graph illustrating the effects of the CMV enhancer on the expression activity of a GFAP promoter;

**FIG. 2** is a graph of results of dose-dependent gene expression of luciferase reporter gene in glioma cells;

**FIG. 3** is a graph depicting the activities of different regulatory elements in the context of baculovirus vectors in glioma cells;

**FIG. 4** is graphs showing the kinetics of luciferase expression in three glioma cell lines infected with recombinant baculoviruses BV-CMV, BV-CMV E/GFAP, and BV-CMV E/GFAP-ITR;

**FIG. 5** is a graph showing luciferase gene expression in rat brains following injection of BV-CMV, BV-GFAP, BV-CMV E/GFAP, and BV-CMV E/GFAP-ITR;

**FIG. 6** is fluorescence photographs showing immunohistological staining of rat brains injected with recombinant BV-CMV E/GFAP-ITR baculovirus;

**FIG. 7** is graphs demonstrating luciferase expression in neurons in a brain region remote (cerebral cortex) from the injection site (striatum);

**FIG. 8** is graphs showing the viability of cells infected with various baculovirus vectors expressing either luciferase or p53;

**FIG. 9** illustrates the results of inoculating a rat brain with and without BV-CMV E/GFAP-p53: **A** is a luminescent scan of the brain and **B** is a graphical representation of a luciferase assay of tissue collected either 3 or 14 days after inoculation;

**FIG. 10** is graphs demonstrating baculovirus-mediated transduction in glioma cells: **A** shows luciferase expression in glioma cells and **B** shows EGFP expression in glioma cells;

**FIG. 11** is graphs demonstrating that modified GFAP promoters have improved baculovirus-mediated transduction in glioma cells: **A** depicts the results using baculoviral vectors with a luciferase reporter gene (schematic diagram of the expression cassettes is shown on the top) and **B** depicts results from the experiment with BV-CMV-EGFP;

**FIG. 12** shows the *in vitro* effects of baculovirus vectors carrying the DT-A gene: **A** is a photograph of a gel of RT-PCR products of DT-A expression in U251 cells 48 h after transduction at MOI of 100, **B** is graphs depicting protein synthesis inhibition, as demonstrated by decrease of luciferase activity in 6 glioma cell lines, **C** is a graph of results using IVIS imaging of transduction of BV-CG/ITR-DTA in C6-Luc cells, and **D** is graph showing cell viability in various cell lines;

**FIG. 13** shows results of *in vivo* transgene expression in gliomas mediated by baculovirus carrying the hybrid CMV E/GFAP promoter and ITRs: **A** is fluorescence photographs showing BV-CG/ITR-EGFP injected into rat striatum, and B is a graph of the quantification of the transgene expression;

**FIG. 14** is results of *in vivo* assays of C6 glioma growth in the rat brain: **A** is *in vivo* bioluminescent images of the brains with inoculated C6-luc cells, **B** is graphs of the quantification of the *in vivo* bioluminescence and **C** is a graph showing measurement of tumour growth by luciferase activity assays of brain tissues;

**FIG. 15** is a schematic diagram of the neuronal specific expression cassettes used in certain baculoviral vectors;

**FIG. 16** is graphs showing the kinetics of luciferase expression in cultured cells infected with recombinant baculoviruses;

**FIG. 17** depicts the results of analysis of baculovirus-infected NT-2 cells: **A** is a Southern blot and **B** is a graph showing the results of real-time PCR of luciferase gene expression;

**FIG. 18** is a graph indicating the kinetics of luciferase expression in rat brain after injection of recombinant baculovirus;

**FIG. 19** is graphs demonstrating PDGF promoter activity in neurons in a brain region remote from the injection site; and

**FIG. 20** is fluorescence photographs of rat brains injected with BV-CMV E/PDGF-ITR or BV-CMV.

### DETAILED DESCRIPTION

A hybrid neural promoter has been developed as described herein, which is particularly suited for expression of a a therapeutic product in neural cells in the ventral nervous system including neuronal cells or glial cells, or cells derived from neural cells, including neurons, neuroblatomas, astrocytes and astrocytoma. The hybrid promoter can be used with a nucleic acid vector, and is particularly useful when included in a baculovirus vector for delivery to and expression of a therapeutic gene in neural cells, including neuronal cells and glial cells.

There is described herein a hybrid neural promoter that is specific for expression in neural cells and in cells derived from neural cells. The present hybrid neural promoter is useful for expressing a coding sequence that encodes an expression product, which product may be a therapeutic expression product, specifically in neural cells or in cells derived from neural cells, including where the neural cells are neuronal cells, or glial cells including astrocytes or cells derived from astrocytes. The present hybrid neural promoter may include a neuronal-specific promoter and is particularly useful for gene therapy of neuronal related disorders, including neuronal cancers, including medulloblastoma. The hybrid neural promoter may alternatively include a glial-specific promoter and is particularly useful for gene therapy of gliomas, including astrocytomas.

The novel promoter as described herein is a hybrid promoter containing regulatory components from neural-specific genes and an enhancer element, including a viral enhancer element. Thus, the promoter comprises a neural-specific promoter and an enhancer element, in one embodiment from a viral promoter, located upstream or downstream of the neural-specific promoter.

As will be understood, a promoter or a promoter region is a nucleotide sequence located upstream of a coding region of a gene that contains at least the minimal necessary DNA elements required to direct transcription of the coding region, and typically includes a site that directs RNA polymerase to the transcription initiation start site and one or more transcription factor binding sites. A promoter, including a native promoter may include a core promoter region, for example containing a TATA box and an initiator sequence, and it may further include a regulatory region containing proximal promoter elements outside of the core promoter that act to enhance or regulate the level of transcription from the core promoter, including enhancer elements normally associated with a given promoter.

As used herein, a "neural cell" refers to a cell or cells found in the central nervous system, and includes neurons and glia, the two main types of neural cells, and cells derived from neurons and glia, including neoplastic and tumour cells derived from neurons or glia. A "cell derived from a neural cell" refers to a cell which is derived from or originates or is differentiated from a neural cell.

"Neural" describes something that is of, related to, or comprises, neural cells. The term "neural-specific" refers to something that is found, or an activity that occurs, in neural cells or in cells derived from neural cells, but is not found in or occur in, or is not found substantially in or occur substantially in, non-neural cells or in cells not derived from neural cells.

A "neural specific promoter" is a promoter that controls expression of genes that are uniquely or predominantly expressed in neural cells or in cells derived from neural cells, including neuronal cells or glial cells. Thus, a neural-specific promoter includes a neuronal-specific promoter and a glial-specific promoter. A neural-specific promoter directs expression of a gene in neural cells or in cells derived from neural cells, but does not substantially direct expression of that same gene in other cell types, thus having neural specific transcriptional activity. In some instances there may be some low level expression in other cell types, but such expression is substantially lower than in neural cells, for example about less than 1% or about 1%, 2%, 3%, 5%, 10%, 15% or 20% of the expression levels in neural cells. Such a promoter may be a strong promoter or it may be a weak promoter, and it may direct constitutive expression of a gene in a neural cell or a cell derived from a neural cell, or it may direct expression in response to certain conditions, signals or cellular events. For example, the promoter may be an inducible promoter that requires a particular ligand, small molecule, transcription factor or hormone protein in order to effect transcription from the promoter.

Neural cells include neuronal cells and glial cells. As used herein, a "neuronal cell" or "neuron" refers to a cell or cells found in the central nervous system which act to transmit nerve impulses, and which typically comprise a soma, a dendrite and an axon. Neuronal cells include unipolar and pseudounipolar neurons, bipolar neurons and multipolar neurons including type I and type II golgi. Neurons typically communicate with adjacent neurons through either chemical or electrical synapses. A "cell derived from a neuronal cell" refers to a cell which is derived from or originates or is differentiated from a neuronal cell, including neoplastic and tumor cells, including neuronal tumour cells and neuronal cancers, including medulloblastomas and neuroblastomas, including benign ganglioneuromas, partially differentiated ganglioneuroblastomas, and malignant neuroblastomas.

"Neuronal" describes something that is of, related to, or comprises, neuronal cells. The term "neuronal-specific" refers to something that is found, or an activity that occurs, in neuronal cells or cells derived from neuronal cells, but is not found in or occur in, or is not found substantially in or occur substantially in, non-neuronal cells or cells not derived from neuronal cells, for example glial cells. For example, when a coding sequence is expressed in neuronal cells and is not substantially expressed in non-neuronal cells, the product of the coding sequence may still be expressed at low levels in the non-neuronal cells, for example at about less than 1% or about 1%, 2%, 3%, 5%, 10%, 15% or 20% of the levels at which the product is expressed in neuronal cells. Similarly, activity such as transcriptional activity that is neuronal-specific may occur at levels of about less than 1% or about 1%, 2%, 3%, 5%, 10%, 15% or 20% of the levels at which the activity occurs in neuronal cells.

Thus, a neuronal-specific promoter is a promoter that controls expression of genes that are uniquely or predominantly expressed in neuronal cells or in cells derived from neuronal cells, including neuronal cancer cells. A neuronal-specific promoter directs expression of a gene in neuronal cells or in cells derived from neuronal cells, but does not substantially direct expression of that same gene in other cell types, for example glial cells, thus having neuronal specific transcriptional activity. In some instances there may be some low level expression in other cell types, but such expression is substantially lower than in neuronal cells, for example about less than 1% or about 1%, 2%, 3%, 5%, 10%, 15% or 20% of the expression levels in neuronal cells. Such a promoter may be a strong promoter or it may be a weak promoter, and it may direct constitutive expression of a gene in a neuronal cell or a cell derived from a neuronal cell, or it may direct expression in response to certain conditions, signals or cellular events. For example, the promoter may be an inducible promoter that requires a particular ligand, small molecule, transcription factor or hormone protein in order to effect transcription from the promoter.

As used herein, a "glial cell" or "glia" refers to a cell or cells found in the central nervous system which act as support for neuronal cells, which do not conduct electrical impulses and which play a role in repair and regeneration of nervous tissue. Glial cells include astro-glial cells or astrocytes, which are star-shaped glial cells and which may be fibrous astrocytes or protoplasmic astrocytes. Protoplasmic astrocytes are found mainly in the grey matter while fibrous astrocytes occur mainly in the white matter of the brain and spinal cord. Glial cells also include microglial cells and either oligodendrocytes (in the central nervous system) or Schwann cells (in the peripheral nervous system). A "cell derived from a glial cell" refers to a cell which is derived from, originated from or is differentiated from a glial cell, including an astrocyte, and includes neoplastic and tumour cells, including gliomas and astrocytomas, including low grade and high grade astrocytomas, and may be any such mammalian cell, including a human cell, a rat cell or a mouse cell.

The term "cell" or "cells" refers to a single cell, as well as a plurality of cells, a culture of cells, a growth of cells, a population of cells or a cell line, and may be *in vitro* or *in vivo,* unless otherwise specified.

"Glial" describes something that is of, related to, or comprises, glial cells. Similarly, "astro-glial" describes something that is of, related to, derived from, or comprises, astro-glial cells or astrocytes. The term "glial-specific" refers to something that is found, or an activity that occurs, in glial cells or in cells derived from glial cells but is not found in or occur in, or is not found substantially in or occur substantially in, non-glial cells or in cells not derived from glial cells, for example neuronal cells. For example, when a coding sequence is ex-pressed in glial cells and is not substantially expressed in non-glial cells, the product of the coding sequence may still be expressed at low levels in the non-glial cells, for example at about less than 1% or about 1%, 2%, 3%, 5%, 10%, 15% or 20% of the levels at which the product is expressed in glial cells. Similarly, activity such as transcriptional activity that is glial-specific may occur at levels of about less than 1% or about 1%, 2%, 3%, 5%, 10%, 15% or 20% of the levels at which the activity occurs in glial cells.

Thus, a glial-specific promoter is a promoter that controls expression of genes that are uniquely or predominantly expressed in glial cells or in cells derived from glial cells, including glioma cells and astrocytes. A glial-specific promoter directs expression of a gene in glial cells or in cells derived from glial cells, but does not substantially direct expression of that same gene in other cell types, for example neuronal cells, thus having glial specific transcriptional activity. In some instances there may be some low level expression in other cell types, but such expression is substantially lower than in glial cells, for example about less than 1% or about 1%, 2%, 3%, 5%, 10%, 15% or 20% of the expression levels in glial cells. Such a promoter may be a strong promoter or it may be a weak promoter, and it may direct constitutive expression of a gene in a glial cell or a cell derived from a glial cell, or it may direct expression in response to certain conditions, signals or cellular events. For example, the promoter may be an inducible promoter that requires a particular ligand, small molecule, transcription factor or hormone protein in order to effect transcription from the promoter.

A neural-specific promoter, including a neuronal-specific promoter and a glial-specific promoter, is used to avoid expression from the hybrid promoter when placed in non-neural derived cells. A neuronal-specific promoter may be used to avoid expression in adjacent glial cells, and similarly, a glial-specific promoter may be used to avoid expression in adjacent neurons or neuron-derived cells. In addition to offering cell-type specificity, these promoters are less likely to activate intracellular defense machinery because of their authentic cellular sequences, thus being less sensitive to promoter silencing as compared to viral promoters (Dressel, U., et al., Anticancer Res (2000) 20:1017-1022).

In certain cases, the neural-specific promoter is a neuronal-specific promoter. Neuronal cell specific promoters may include promoters for neuronal genes such as Synapsin I, Neuron-specific enolase, Neurofilament-L and Neuropeptide Y and promoters specific for particular types of neuronal cells. For example, tyrosine hydroxylase gene promoter (4.8kb 5' UTR) is specific for catecholaminergic and the CNS neurons, dopamine-b-hydroxylase gene promoter is specific for adrenergic and noradrenegic neurons and L7 Purkinje cell protein promoter is specific for retinal rod bipolar neurons. For these and other neuronal cell specific promoters including, D1A dopamine receptor gene promoter, human hypoxanthine phosphoribosyltransferase promoter, SCG10 promoter, Tα1 α-tubulin promoter, aldolase C promoter, beta-tubulin gene promoter, GnRH gene enhancer and promoter, glutamate decarboxylase 65 gene promoter, beta-galactoside alpha1,2-fucosyltransferase gene promoter, neuronal nicotinic acetylcholine receptor beta3 gene promoter, GABA(A) receptor delta subunit gene promoter, neuron-specific FE65 gene promoter, N-type calcium channel alpha1B subunit gene promoter and microtubule-associated protein 1B gene promoters, see Harrington CA, Lewis EJ, Krzemien D, Chikaraishi DM. Identification and cell type specificity of the tyrosine hydroxylase gene promoter.Nucleic Acids Res 1987, 15:2363-2384; Coker GT 3rd, Vinnedge L, O'Malley KL; Characterization of rat and human tyrosine hydroxylase genes: functional expression of both promoters in neuronal and non-neuronal cell types. Biochem Biophys Res Commun 1988, 157:1341-1347; Banerjee SA, Hoppe P, Brilliant M, Chikaraishi DM. 5' flanking sequences of the rat tyrosine hydroxylase gene target accurate tissue-specific, developmental, and transsynaptic expression in transgenic mice. J Neurosci 1992, 12:4460-4467; Morita S, Kobayashi K, Mizuguchi T, Yamada K, Nagatsu I, Titani K, Fujita K, Hidaka H, Nagatsu T. The 5'-flanking region of the human dopamine beta-hydroxylase gene promotes neuron subtype-specific gene expression in the central nervous system of transgenic mice. Brain Res Mol Brain Res 1993; 17:239-244; Ishiguro H, Kim KT, Joh TH, Kim KS. Neuron-specific expression of the human dopamine beta-hydroxylase gene requires both the cAMP-response element and a silencer region. J Biol Chem 1993; 268:17987-17994; Hoyle GW, Mercer EH, Palmiter RD, Brinster RL. Cell-specific expression from the human dopamine beta-hydroxylase promoter in transgenic mice is controlled via a combination of positive and negative regulatory elements. J Neurosci 1994, 14:2455-2463; Severynse DM, Colapietro AM, Box TL, Caron MG. The human D1A dopamine receptor gene promoter directs expression of a reporter gene to the central nervous system in transgenic mice. Brain Res Mol Brain Res 1995, 30:336-346; Mouradian MM, Minowa MT, Minowa T. Promoter structure of the human gene coding for the D1A dopamine receptor. Adv Neurol 1993, 60:343-345; Stout JT, Chen HY, Brennand J, Caskey CT, Brinster RL. Expression of human HPRT in the central nervous system of transgenic mice. Nature 1985; 317:250-252; Rincon-Limas DE, Geske RS, Xue JJ, Hsu CY, Overbeek PA, Patel PI. 5'-flanking sequences of the human HPRT gene direct neuronal expression in the brain of transgenic mice. J Neurosci Res 1994; 38:259-267; Schwartz ML, Bruce J, Shneidman PS, Schlaepfer WW. Deletion of 3'-untranslated region alters the level of mRNA expression of a neurofilament light subunit transgene. J Biol Chem 1995; 270:26364-9; Forss-Petter S, Danielson PE, Catsicas S, Battenberg E, Price J, Nerenberg M, Sutcliffe JG. Transgenic mice expressing beta-galactosidase in mature neurons under neuron-specific enolase promoter control. Neuron 1990; 5:187-197; Twyman RM, Jones EA. Sequences in the proximal 5' flanking region of the rat neuron-specific enolase (NSE) gene are sufficient for cell type-specific reporter gene expression. J Mol Neurosci 1997; 8:63-73; Andersen JK, Garber DA, Meaney CA, Breakefield XO. Gene transfer into mammalian central nervous system using herpes virus vectors: extended expression of bacterial lacZ in neurons using the neuron-specific enolase promoter. Hum Gene Ther 1992; 3:487-499; Wuenschell CW, Mori N, Anderson DJ. Analysis of SCG10 gene expression in transgenic mice reveals that neural specificity is achieved through selective derepression. Neuron 1990; 4:595-602; Mori N, Stein R, Sigmund O, Anderson DJ. A cell type-preferred silencer element that controls the neural-specific expression of the SCG10 gene. Neuron 1990; 4:583-594; Hoesche C, Sauerwald A, Veh RW, Krippl B, Kilimann MW. The 5'-flanking region of the rat synapsin I gene directs neuron-specific and developmentally regulated reporter gene expression in transgenic mice. J Biol Chem 1993; 268:26494-26502; Kilic E, Hermann DM, Kugler S, Kilic U, Holzmuller H, Schmeer C, Bahr M. Adenovirus-mediated Bcl-X(L) expression using a neuron-specific synapsin-1 promoter protects against disseminated neuronal injury and brain infarction following focal cerebral ischemia in mice. Neurobiol Dis 2002; 11:275-284; Gloster A, Wu W, Speelman A, Weiss S, Causing C, Pozniak C, Reynolds B, Chang E, Toma JG, Miller FD. The T alpha 1 alpha-tubulin promoter specifies gene expression as a function of neuronal growth and regeneration in transgenic mice. J Neurosci 1994; 14:7319-7330; Thomas M, Makeh I, Briand P, Kahn A, Skala H. Determinants of the brain-specific expression of the rat aldolase C gene: ex vivo and in vivo analysis. Eur J Biochem 1993; 218:143-151; Thomas M, Skala H, Kahn A, Tuy FP. Functional dissection of the brain-specific rat aldolase C gene promoter in transgenic mice. Essential role of two GC-rich boxes and an HNF3 binding site. J Biol Chem 1995; 270:20316-20321; Dennis K, Uittenbogaard M, Chiaramello A, Moody SA. Cloning and characterization of the 5'-flanking region of the rat neuron-specific Class III beta-tubulin gene. Gene 2002 294:269-277; Waldbieser GC, Minth CD, Chrisman CL, Dixon JE. Tissue-specific expression of the human neuropeptide Y gene in transgenic mice. Brain Res Mol Brain Res 1992; 14:87-93; Lawson MA, Macconell LA, Kim J, Powl BT, Nelson SB, Mellon PL. Neuron-specific expression in vivo by defined transcription regulatory elements of the GnRH gene. Endocrinology 2002; 143:1404-1412; Wolfe A, Kim HH, Tobet S, Stafford DE, Radovick S. Identification of a discrete promoter region of the human GnRH gene that is sufficient for directing neuron-specific expression: a role for POU homeodomain transcription factors. Mol Endocrinol 2002; 16:435-449; Makinae K, Kobayashi T, Kobayashi T, Shinkawa H, Sakagami H, Kondo H, Tashiro F, Miyazaki J, Obata K, Tamura S, Yanagawa Y. Structure of the mouse glutamate decarboxylase 65 gene and its promoter: preferential expression of its promoter in the GABAergic neurons of transgenic mice. J Neurochem 2000; 75:1429-14371; Hitoshi S, Kusunoki S, Kanazawa I, Tsuji S. Dorsal root ganglia neuron-specific promoter activity of the rabbit beta-galactoside alpha1,2-fucosyltransferase gene. J Biol Chem 1999; 274:389-396; Roztocil T, Matter-Sadzinski L, Gomez M, Ballivet M, Matter JM. Functional properties of the neuronal nicotinic acetylcholine receptor beta3 promoter in the developing central nervous system. J Biol Chem 1998; 273:15131-15137; Luscher B, Hauselmann R, Leitgeb S, Rulicke T, Fritschy JM. Neuronal subtype-specific expression directed by the GABA(A) receptor delta subunit gene promoter/upstream region in transgenic mice and in cultured cells. Brain Res Mol Brain Res 1997; 51:197-211; Zambrano N, De Renzis S, Minopoli G, Faraonio R, Donini V, Scaloni A, Cimino F, Russo T. DNA-binding protein Pur alpha and transcription factor YY1 function as transcription activators of the neuron-specific FE65 gene promoter. Biochem J 1997; 328:293-300; Kim DS, Jung HH, Park SH, Chin H. Isolation and characterization of the 5'-upstream region of the human N-type calcium channel alpha1B subunit gene. Chromosomal localization and promoter analysis. J Biol Chem 1997; 272:5098-5104 and Liu D, Fischer I. Two alternative promoters direct neuron-specific expression of the rat microtubule-associated protein 1B gene. J Neurosci 1996; 16:5026-5036. Other neuronal specific promoters will be known to persons skilled in the art.

The neuronal-specific promoter may be the promoter from platelet-derived growth factor β-chain (PDGF), and in certain embodiments is the promoter from human PDGF. The PDGF promoter (Sasahara M, Fries JW, Raines EW, Gown AM, Westrum LE, Frosch MP, Bonthron DT, Ross R, Collins T. PDGF β-chain in neurons of the central nervous system, posterior pituitary, and in a transgenic model. Cell 1991; 64:217-227) has been shown to be specific for CNS neuronal cells, including dopaminergic neurons.

The neuronal cell specific promoter may comprise the following sequence [SEQ ID NO: 10] representing nucleotides -1492 to -5 from the transcription start site of the human PDGF β gene, as described in PCT publication WO2004087926 :

The neural-specific promoter is a glial-specific promoter.

The glial-specific promoter may include the JC virus early promoter (Kim, S.Y., et al., J Virol. (2003) 77:3394-401), the myelin basic protein promoter (Wei, Q., et al., Gene. (2003) 313:161-7), or the S100beta promoter (Namihira, M., et al., FEBS Lett. (2004) 572:184-8).

The glial-specific promoter may include the promoter region of glial fibrillary acidic protein (GFAP) gene, which is specific to astro-glial cells and cells derived from same. The glial-specific promoter region may have the sequence of the human GFAP promoter or the rat GFAP promoter, as shown below:

Human GFAP promoter [**SEQ ID NO: 1**]:

Rat GFAP promoter [**SEQ ID NO: 2**]:

In addition to cell-type specificity, high-level expression of therapeutic genes is desirable for cancer gene therapy. Compared to other cellular promoters, GFAP promoters possess a relatively strong activity, capable of driving expression of a transgene representing up to approximately 0.2% of total brain protein (Smith et al., Neurobiol Aging. (1998) Sep-Oct;19(5):407-13).

However, like many other cellular promoters, GFAP promoters display a relatively weak transcriptional activity when compared to widely used strong promoters derived from viruses, for example the cytomegalovirus immediately early promoter and enhancer (CMV promoter, Morelli et al., J Gen Virol. (1999) Mar;80 ( Pt 3):571-83; Biglari et al., Cancer Gene Ther. (2004) Nov;11(11):721-32). This inherent weakness in driving gene expression might affect efficacy of cancer gene therapy applications in the CNS that require a high level expression of a therapeutic gene (Biglari et al., (2004), *supra*).

In order to increase the levels of transcription, the present hybrid promoter includes a heterologous enhancer element operably linked to the neural-specific, including neuronal-specific or glial-specific, promoter. The heterologous enhancer element is any enhancer element that is not normally associated with the neural-specific promoter and may be a viral enhancer element, or it may be an enhancer element from a cellular gene, provided that the enhancer functions to enhance transcriptional activity of the neural-specific promoter when included in the hybrid promoter as described herein.

As will be understood, an enhancer or an enhancer element is a cis-acting sequence that increases the level of transcription of a promoter, and can function in either orientation relative to the promoter and the coding sequence that is to be transcribed, and can be located upstream or downstream relative to the promoter or the coding region of a gene.

Generally, enhancers act to increase and/or activate transcription from an operably linked promoter once bound by appropriate molecules such as transcription factors. For various enhancers which may be used, transcription factor binding sites may be known or identified by one of ordinary skill using methods known in the art, for example by DNA footprinting, gel mobility shift assays, and the like. The factors may also be predicted on the basis of known consensus sequence motifs.

Reference to increasing the transcription levels or transcriptional activity is meant to refer to any detectable increase in the level of transcription of operably linked sequences compared to the level of the transcription observed with a neural-specific promoter alone, as may be detected in standard transcriptional assays, including using a reporter gene construct as described in the Examples set out below.

A first nucleic acid sequence is operably linked with a second nucleic acid sequence when the sequences are placed in a functional relationship. For example, a coding sequence is operably linked to a promoter if the promoter activates the transcription of the coding sequence. Similarly, a neural-specific promoter, including a neuronal-specific promoter or a glial-specific promoter, and an enhancer element, including a viral enhancer, are operably linked when the enhancer increases the neural-specific transcription of operably linked sequences. Operably linked sequences may be contiguous. However, enhancers may function when separated from promoters and thus a heterologous enhancer may be operably linked to a neural-specific promoter but may not be contiguous with that promoter.

The placement of the heterologous enhancer relative to a neural-specific promoter may vary in location, orientation and/or number, leading to hybrid promoters with varying degrees of activities. The level of transcriptional activity of any given hybrid promoter can be readily tested using standard methods in the art, including transcription of a reporter gene from the hybrid promoter and measurement of the level of the reporter gene product, for example using the β-luciferase gene. The heterologous enhancer element may be placed immediately upstream to, and contiguous with, the neural-specific promoter.

The heterologous enhancer element may include a cellular enhancer, for example the Alpha-Fetoprotein (AFP) enhancer or the tyrosinase enhancer.

The heterologous enhancer element may include a viral enhancer element, including the cytomegalovirus (CMV) immediate early enhancer element and the SV40 enhancer element. The region located at -568 to -187 base pairs relative to the TATA box of the CMV immediate early promoter may used as the viral enhancer. The viral enhancer element may single CMV enhancer element.

Thus, the heterologous enhancer may have the following sequence [SEQ ID NO: 3], as published under genebank accession number K03104:

The hybrid promoter may comprise the CMV enhancer upstream of, contiguous with, and operably linked to, the GFAP promoter. The hybrid promoter may comprise the CMV enhancer upstream of, contiguous with, and operably linked to, the PDGF promoter.

The hybrid promoter may include allelic variants and derivatives of neural-specific promoters, which may be operably linked to allelic variants and derivatives of a heterologous enhancer element, provided such variants and derivatives act to increase transcriptional activity of the hybrid promoter beyond the native levels of the particular neural-specific promoter included in the hybrid promoter. Allelic variants and derivatives include deletions, insertions, inversion, substitutions or addition of sequences.

Such variants and derivatives may be substantially homologous to an endogenous neural-specific promoter or a wildtype or known heterologous enhancer such as a viral enhancer in that the variant or derivative hybridizes to the known promoter and/or enhancer sequence under moderately or highly stringent conditions. Hybridization to filter-bound sequences under moderately stringent conditions may, for example, be performed in 0.5 M NaHPO₄, 7% sodium dodecyl sulfate (SDS), 1 mM EDTA at 65°C, and washing in 0.2 x SSC/0.1% SDS at 42°C (see Ausubel, et al. (eds), 1989, Current Protocols in Molecular Biology, Vol. 1, Green Publishing Associates, Inc., and John Wiley & Sons, Inc., New York, at p. 2.10.3). Alternatively, hybridization to filter-bound sequences under highly stringent conditions may, for example, be performed in 0.5 M NaHPO₄, 7% SDS, 1 mM EDTA at 65°C, and washing in 0.1 x SSC/0.1% SDS at 68°C (see Ausubel, *et al.* (eds), 1989, *supra*). Hybridization conditions may be modified in accordance with known methods depending on the sequence of interest (see Tijssen, 1993, Laboratory Techniques in Biochemistry and Molecular Biology -- Hybridization with Nucleic Acid Probes, Part I, Chapter 2 "Overview of principles of hybridization and the strategy of nucleic acid probe assays", Elsevier, New York). Generally, stringent conditions are selected to be about 5°C lower than the thermal melting point for the specific sequence at a defined ionic strength and pH. Stringent hybridization may for example be in 5 times SSC and 50% formamide at 42 degrees Celcius and washing in a wash buffer consisting of 0.1.times SSC at 65 degrees Celcius. Washes for stringent hybridization may for example be of at least 15 minutes, 30 minutes, 45 minutes, 60 minutes, 75 minutes, 90 minutes, 105 minutes or 120 minutes.

The degree of homology between sequences may also be expressed as a percentage of identity when the sequences are optimally aligned, meaning the occurrence of exact matches between the sequences. Optimal alignment of sequences for comparisons of identity may be conducted using a variety of algorithms, such as the local homology algorithm of Smith and Waterman,1981, Adv. Appl. Math 2: 482, the homology alignment algorithm of Needleman and Wunsch, 1970, J. Mol. Biol. 48:443, the search for similarity method of Pearson and Lipman, 1988, Proc. Natl. Acad. Sci. USA 85: 2444, and the computerised implementations of these algorithms (such as GAP, BESTFIT, FASTA and TFASTA in the Wisconsin Genetics Software Package, Genetics Computer Group, Madison, WI, U.S.A.). Sequence alignment may also be carried out using the BLAST algorithm, described in Altschul et al., 1990, J. Mol. Biol. 215:403-10 (using the published default settings). Software for performing BLAST analysis may be available through the National Center for Biotechnology Information (through the internet at http://www.ncbi.nlm.nih.gov/). In various embodiments, the variants and derivatives may be greater than 50%, 80% to 100%, at least 80%, at least 90% or at least 95% identical as determined using such algorithms.

The mechanism underlying the enhancement of a native neural-specific promoter by inclusion of a heterologous enhancer such as a viral enhancer is not clearly understood yet. Without being limited to a single theory, it is possible that transcriptional factors and auxiliary proteins attracted by a viral enhancer, for example the CMV enhancer, may interact with those attracted by the neural-specific promoter to generate a synergistic action that favours gene expression (Khachigian et al., J Clin Invest (1995) 96:1169-1175; Rafty et al., J Biol Chem (1998) 273:5758-5764).

The above-described hybrid promoter can be synthesized using standard molecular biology and molecular cloning techniques known in the art, for example, as described in Sambrook et al. (2001) Molecular Cloning: a Laboratory Manual, 3rd ed., Cold Spring Harbour Laboratory Press). As will be understood, the term "recombinant" when referring to a nucleic acid molecule or construct means that heterologous nucleic acid sequences have been recombined, such that reference to a recombinant nucleic acid molecule refers to a molecule that is comprised of nucleic acid sequences that are joined together or produced by means of molecular biological techniques.

The above-described hybrid promoter is useful for the expression of an operably linked coding sequence in neural cells, including in neuronal or glial cells or cells derived from neuronal or glial cells as described herein, particularly within a cell as part of a gene therapy treatment. The hybrid promoter may be used in an expression cassette to effect the expression of an operably linked coding region in a neural cell or in a cell derived from a neural cell. Thus, there is provided a nucleic acid molecule that is an expression cassette and which includes the present hybrid promoter operably linked to a coding sequence of interest, or which is capable of being operably linked to a coding sequence of interest, for example by inclusion of a cloning site.

An "expression cassette" refers to a nucleic acid molecule, generated recombinantly or synthetically, which contains appropriate regulatory regions, including a promoter and transcription termination sequences, which permit transcription of an operably linked coding sequence in an appropriate expression system. The expression cassette may include the regulatory sequences linked to a cloning site, for example a multiple cloning site, into which a coding sequence may be inserted so as to become operably linked to the regulatory regions. Alternatively, the expression cassette may already include the coding sequence operably linked to the regulatory regions. In another alternative, the expression cassette may include the coding sequence operably linked to the regulatory regions and a cloning site also operably linked to the regulatory regions to enable synthesis of an expression cassette for a fusion product. Thus, the expression cassette may include one or both of the coding sequence or the cloning site, each operably linked to the regulatory regions. If the expression cassette includes both the coding sequence and the cloning site, the cloning site may be upstream or downstream of the coding sequence.

The expression cassette may be incorporated into a larger nucleic acid molecule for delivery to and stability in an expression system, for example, the expression cassette may be included in a plasmid, chromosome, mitochondrial DNA, plastid DNA, virus, or nucleic acid fragment.

A coding sequence of interest is a nucleic acid sequence that encodes any molecule that is desired to be expressed under control of the present hybrid promoter in neural cells or cells derived from neural cells, including in neuronal or glial cells or in cells derived from neuronal or glial cells. The coding sequence may encode a protein, a peptide, a ribozyme, a small interfering RNA (siRNA), a microRNA or an antisense RNA. The coding sequence may or may not include downstream regulatory regions, for example a polyadenylation signal.

The expression cassette may further include viral inverted terminal repeat sequences ("ITRs") flanking the region encompassing the hybrid promoter, the operably linked coding sequence including any downstream regulatory regions, or cloning site. It will be understood that when the enhancer element is located remote from the neural-specific promoter, the ITRs may flank the neural-specific promoter, the operably linked coding sequence or cloning site and any downstream regulatory regions, with the enhancer element located outside the ITRs. By "flanking" it is meant that a pair of ITRs is placed on either side, with one ITR sequence upstream and one ITR sequence downstream, of the relevant nucleotide sequences. In a particular embodiment, the present expression cassette includes ITRs from Adeno-associated virus ("AAV").

Inverted terminal repeats (ITRs) are AAV elements, serving as a primer for host-cell mediated DNA synthesis to convert single-stranded viral DNA to a double-strand DNA template for transcription and replication (McCarty et al., Annu Rev Genet. (2004) 38: 819-45). A number of reports also suggested their roles in mediating a substrate DNA integration into the host DNA (Xiao et al., J Virol. (1997) 71:941-8; Yang et al., J Virol. (1997) 71:9231-47) and in defining integration boundaries of the viral genome (Philpott et al., Proc Natl Acad Sci U S A. (2002) 99:12381-5). The presence of ITRs appears the only requirement for the formation of episomally stable concatamers of recombinant AAV genome that occur through intermolecular recombination between two independent linear and/or circular genome (Duan et al., J Virol. (1998) 72:8568-77; Yang et al., J Virol. (1999) 73:9468-77). Interestingly, long-term transgene expression mediated by AAV vectors was found to be associated with the molecular conversion of single-stranded viral genomes to high-molecular-weight circular concatamers and prolonged episomal persistence of the concatamers, with a head-to-tail ITR DNA element contained within the circular concatamers being responsible for mediating the increased persistence of transgene expression (Duan et al., 1998, *supra*).

Inclusion of AAV ITRs that flank expression cassettes comprising a native cellular promoter can result in loss of cellular specificity. It has been reported that in the context of AAV carrying a GFAP promoter, most transduced brain cells appeared neuronal (Peel and Klein, J Neurosci Methods (2000) Jun 1;98(2):95-104; Xu, R. et al., Gene Ther. (2001) Sep;8(17):1323-32) rather than astro-glial derived. In some instances the AAV ITR sequences appear to function directly as a promoter for expression of a reporter gene (Flotte et al., J Biol Chem. (1993) 268:3781-90), overriding the GFAP promoter. This mechanism has been suspected to function as the possible mechanism underlying the neuronal expression of genes under control of the GFAP promoter when flanked by AAV ITRs (Fitzsimons et al., Methods. (2002) 2002 Oct;28(2):227-36).

However, with the promoter and the various constructs and methods as described herein, it has been surprisingly discovered that inclusion of the ITRs does not alter the cell specificity of expression from the neural-specific promoter combined with the heterologous enhancer, including when the neural-specific promoter is a neuronal-specific or glial-specific promoter.

The ITRs may have the following sequence [**SEQ ID NO: 4**], as published under accession numbers J01901, M12405, M12468 and M12469:

The ITRs may be oriented in the same orientation as in wild-type AAV-2, with the upstream ITR sequence located immediately adjacent and upstream to the promoter on one-side and the downstream ITR sequence located immediately adjacent to and downstream to the polyA sequence of the coding region.

Incorporation of ITRs such as AAV ITRs in an expression cassette including the present hybrid promoter further increases expression levels of an operably linked coding sequence. Inclusion of ITRs appears not to change the dynamics of transgene expression. With or without ITRs, expression from an expression cassette driven by the present hybrid promoter display similar patterns of time-dependent decrease in gene expression both *in vitro* and *in vivo.* Nevertheless; collaborative action of AAV ITRs and the hybrid promoter augment initial levels of trans gene expression.

The expression cassette may comprise the sequence of **SEQ ID NO: 5** upstream of the operably-linked coding sequence and the sequence of **SEQ ID NO: 6** downstream of the operably-linked coding sequence.

[**SEQ ID NO: 5**]:

[**SEQ ID NO: 6**]:

The expression cassette described herein may be readily constructed using known molecular biology and cloning methods, as described above. The present expression cassette is useful for insertion in an expression vector for use in an appropriate expression system that can support transcription from a neural-specific promoter.

Described herein is an expression vector comprising the present hybrid promoter and/or the present expression cassette. As stated above, the expression vector may be a plasmid, a chromosome including an artificial chromosome, a mitochondrial DNA, a plastid DNA, a virus, or a nucleic acid fragment.

The expression vector may be a viral vector. Baculovirus vectors are particularly suited for expression of a transgene in neuronal cells, or in glial cells, particularly astrocytes and cells derived from astrocytes.

Baculovirus infection does not lead to expression of its own genes or viral replication in mammalian cells (Carbonell et al., J Virol. (1985) Oct;56(1):153-60; Hofmann et al., Proc Natl Acad Sci USA (1995) 92: 10099-10103; Stanbridge et al., J Biomed Biotechnol. (2003) 2003(2):79-91). As a result, even though certain sequences of the virus could function as promoters or enhancer of transcription, they will be silent in mammalian cells due to the absence of supporting factors and less likely to influence the cell-type specificity of a mammalian promoter inserted into a baculovirus vector.

Thus, the viral vector may be baculovirus *Autographa californica multiple nucleopolyhedrovirus* (AcMNPV). AcMNPV-based vectors are emerging as a new generation of gene therapy vehicles (Hofmann et al., (1995), *supra;* Boyce et al., Proc Natl Acad Sci USA (1996) 93: 2348-2352; Sarkis et al., Proc. Natl. Acad. Sci. U. S. A. (2000) 97: 14638-43; Ghosh et al., Mol Ther (2002) 6: 5-11; Kost and Condreay, Trends Biotechnol (2002) 20: 173-180; Kost TA, et al., Nat Biotechnol. (2005) 23:567-75).

Baculoviruses are unable to productively replicate and express viral proteins in mammalian cells, meaning these viruses can enter but not replicate in mammalian cells, thus significantly reducing the chance of pre-existing antiviral humoral and cellular immunity in mammals. Other intrinsic advantages that have made AcMNPV an attractive option as a gene delivery vector include a broad tropism for both proliferating and non-proliferating cells, the lack of obvious cytopathic effects, large cloning capacity and easy preparation of high titers of viruses.

Used as a gene vector for systemic delivery, baculoviruses are inactivated easily after exposed to serum complements (Hofmann et al., Gene Ther (1998) 5: 531-536). The central nervous system (CNS), protected by blood-brain barrier (BBB), is virtually isolated from circulating immunological factors including complement components (Carson and Sutcliffe, J Neurosci Res (1999) 55: 1-8), serving as a suitable organ for baculovirus-mediated gene expression. Direct injection of baculovirus vectors to the brain, using a thin needle and slow injection speed to avoid hemorrhage, usually gives satisfactory levels of transgene expression in the organ (Sarkis et al., (2000), *supra;* Li et al., Mol Ther. (2004) 10:1121-9; Li et al. Exp Physiol. (2005) 90:39-44). Attractively, baculoviruses display a high tropism for glial cells (Sarkis et al., (2000), *supra*). A previous study using Cy3-labled baculoviruses demonstrated that about 70% of virus-infected cells in the striatum were glial cells (Li et al., (2004), *supra*). Due to this factor in combination with the present hybrid promoter which incorporates a glial-specific promoter, expression of the coding region of interest should be predominantly limited to glial cells, thereby minimizing potential side effects on important functional neurons that could otherwise be elicited by expression of exogenous genes. Predominantly limited to glial cells means that the expression is substantially lower in non-glial cells, as described above.

A skilled person will be able to construct a suitable vector, including a viral vector and particularly a baculovirus vector, using known molecular biology and cloning techniques, and be able to test the ability of the constructed vector to deliver the hybrid promoter and/or expression cassette, including an operably linked coding sequence, to a neural cell or cell derived from same, including a neuronal or glial cell or a neuronal cell- or glial cell-derived cell. Similarly, a skilled person will be able to determine whether a particular viral vector influences the cellular specificity of expression from the present hybrid promoter using routine testing, for example, by monitoring expression of a reporter gene using various vector constructs in different cell types.

In particular, baculoviruses are well known and characterized and baculoviral vectors for mammalian gene transfection, including the use of *Autographa Californica* are known (see for example, Sarkis, C. et al. (2000) Proc Nati Acad Sci 97(26):14638-43). A skilled person can readily construct any suitable baculoviral vector for use in this invention. The baculovirus may be so modified using standard techniques that will be known to a skilled person, such as PCR and molecular cloning techniques. For example, baculovirus can be readily modified using commercially available cloning and expression systems such as the Bac-To-Bac^{™} Baculovirus Expression system (Gibco BRL, Life Technologies, USA).

The coding sequence operably linked to the present hybrid promoter, included in the present expression cassette, and/or included in the expression vector, and which is to be expressed in a neural cell, neuronal cell or glial cell or in a cell derived from a neural cell, neuronal cell or glial cell, may encode a therapeutic product. The term "therapeutic product" refers to any expression product encoded by the coding sequence, the expression of which effects a desired result, for example, treatment, prevention or amelioration of a neural-related disease or disorder.

A neural-related disease or disorder is any disease or disorder that results from or relates to a defect, genetic mutation, dysfunction or abnormality of a neural cell or a cell-derived from a neural cell.

A neuronal-related disease or disorder is any disease or disorder that results from or relates to a defect, genetic mutation, dysfunction or abnormality of a neuronal cell or a cell-derived from a neuronal cell. Examples of neuronal-related diseases or disorders include neurodegenerative disorders such as Alzheimer's and Parkinson's disease which may be treated for example with a therapeutic gene, for example GDNF (glial-derived neurotrophic factor) or tyrosine hydroxylase. Other examples include stroke, ischemia, epilepsy, head and spinal cord trauma, Parkinson's diseases, Huntington's disease, Alzheimer's disease, amyotrophic lateral sclerosis, and neurogenetic disorders. Neuronal-related diseases or disorders also include neuronal cancers, medulloblastomas and neuroblastomas.

A glial-related disease or disorder is any disease or disorder that results from or relates to a defect, genetic mutation, dysfunction or abnormality of a glial cell or a cell-derived from a glial cell. Examples of glial-related diseases or disorders also include neurodegenerative disorders such as Alzheimer's and Parkinson's disease, which may be treated for example with a therapeutic gene, for example GDNF (glial-derived neurotrophic factor) or tyrosine hydroxylase. Glial cells, and in particular astrocytes, play crucial roles in supporting the survival and physiological functions of neurons. The importance of using these cells as gene transfer targets to express molecules of therapeutic interest in the CNS for the treatment of neuronal diseases and disorders has recently been demonstrated and discussed (Bohn, MC, (2004) Exp Neurol 190: 263-275; Do Thi Na et al., (2004) Gene Ther 11: 746-756; Zhao, Z et al. (2004) Exp Neurol 190: 356-372). Thus, the therapeutic product may be a neurotrophic factor that is secreted by astrocytes to act locally on and support the function of nearby neurons, in a manner similar to neurotrophic factors that are produced in a peripheral target region and act through the classical mechanism of retrograde transport, since under pathological conditions, axonal transport may be hampered or even totally blocked, preventing delivery of neurotrophic factors from remote sites. Gene transfer into glial cells, particularly astrocytes in close proximity to neuronal perikarya does not require axonal transport for neurotrophic factors to reach cell soma and the produced trophic factors may function in a paracrine mode to sustain the survival of the neurons, slow down or stop the degeneration processes of concerned axons, and possibly also stimulate axonal regeneration. Moreover, gene transfer into astrocytes with neurotrophic factors might reduce gliosis, benefiting astrocytes-neuronal cell-cell interactions.

The therapeutic product includes any expression product having clinical usefulness, such as a gene product or protein that is involved in disease prevention or treatment, or a gene product or protein that has a cell regulatory effect that is involved in disease prevention or treatment. The therapeutic product may be a protein, a peptide, a ribozyme, an siRNA, an antisense RNA or a microRNA.

The therapeutic product may directly target the expression of a particular gene required for cell growth or survival. For example, the therapeutic product may be an antisense RNA directed against the telomerase transcript.

The therapeutic product may include a therapeutic protein or peptide. The therapeutic protein or peptide may substitute a defective or missing gene product, protein, or cell regulatory effect in the subject, thereby enabling prevention or treatment of a disease or condition in the subject. Therapeutic proteins and peptides include neurotrophic factors, growth factors (which include the fibroblast growth factor family, nerve growth factor family and insulin-like growth factors), anti-apoptotic proteins (including members of the bcl-2 family), gene products that induce direct killing of the transfected or infected neural cell (including HSV-tk, cytotoxins, tumor suppressor proteins such as p53, p51 or p71, apoptosis-inducers), immunomodulation proteins (such as IL-2, IL-4, IL-12, IFN and TNF-α), angiogenesis inhibitor proteins, tumour suppressor proteins, oncoproteins, suicide proteins, apoptosis proteins, anti-angiogenic proteins and antibodies (including functional fragments of antibodies). The therapeutic product may also be the bacterial DT-A protein.

The therapeutic product may include the p53 protein or a protein in the p53 apoptosis pathway ("p53 pathway protein"). A p53 pathway protein is a protein located in the p53 apoptosis pathway that is involved in effecting or inducing apoptosis in a cell and may be upstream or downstream of p53 in the p53 apoptosis pathway. For example, the p53 pathway protein may be a protein that activates p53, that regulates p53 activity or expression, that is activated by p53 or that is expressed as a result of p53 activation, and that is involved in the apoptosis response related to p53 activation.

Over-expression of the wild type p53 gene is a strategy to either inhibit the growth of tumour cells or promote death of the cells by apoptosis, based on the observations that uncontrolled growth of many tumours results, at least in part, from the loss or mutation of the p53 gene. Restoring normal wild-type p53 function may also enhance apoptotic actions of radiotherapy and chemotherapy, when given in combination with such treatments. Several viral vectors have been tested for p53 gene delivery. One recent study reported the use of baculovirus vectors carrying the p53 gene under the control of the CMV promoter to kill Saos-2 tumour cells (Song et al., Exp Mol Med. (2001) Mar 31;33(1):46-53). Using a hybrid CMV E/GFAP promoter, the current study demonstrated that the glioma cells U251 and C6 could be induced to death with baculovirus vectors expressing p53 from the hybrid promoter. As the hybrid CMV E/GFAP promoter has high glial-specificity, it would be superior to the viral CMV promoter or the original GFAP promoter when applied for gene delivery to the CNS for glioma gene therapy, resulting in less unintended transduction of the p53 gene and causing less or no side effects on neurons. Furthermore, due to the strong transcriptional activity of the hybrid promoter, it is possible that application of a relatively low dose of viruses would be sufficient to offer the threshold level of p53 expression in tumour cells, therefore reducing the possibility of triggering strong immunological reactions due to the use of high doses of viruses.

The therapeutic product may be the DT-A protein. Under the control of a cell-type or tumor specific promoter, the DT-A gene has been tested in cancer therapy (Massuda, E.S., et al. Proc Natl Acad Sci USA (1997) 94:14701-6). This bacterial protein is highly toxic when introduced into the cytoplasm of eukaryotic cells and inhibits protein synthesis by catalyzing ADP ribosylation of the diphthamide group of cellular elongation factor 2 and kills cells through an apoptosis pathway (Michl, P. and Gress, T.M. Curr Cancer Drug Targets (2004) 4:689-702).

The various aspects described herein, including the present hybrid promoter, the present expression cassette and present expression vector, are all useful for the expression of an exogenous expression product, including a therapeutic product, in a neural cell, a neuronal cell, a glial cell or in a cell derived from a neural cell, a neuronal cell or a glial cell, for example, a medulloblastoma tumour cell, an astrocytoma tumour cell or a tissue culture line derived from astrocytes or astrocytoma tumour cells.

Thus, there is described herein a method for expressing an expression product in a neural cell, or in a cell derived from a neural cell.

A neural cell or a cell derived from a neural cell is transfected with a nucleic acid molecule, for example an expression vector, containing a coding sequence encoding the expression product, including a therapeutic product or a product that may act as a reporter molecule, operably linked to a hybrid promoter comprising a neural-specific promoter and a viral enhancer.

The method of transfection will depend on the nature of the cell that is to be transfected, the method of culturing or growing the cell that is to be transfected, as well as the particular expression vector that is to be used.

Standard transfection methods for transfection of mammalian cells with nucleic acid molecules are known, and include transfection with carrier molecules such as liposomes, cationic polymers, cationic lipids and calcium phosphate, transfection by microinjection, needle-free injection, electroporation and using naked DNA.

Where the expression vector is a viral vector, the transfection may be achieved by exposing the cell to a virus particle containing the particular viral expression vector DNA under conditions which allow for infection of the cell by the virus, for example, by addition of live virus to culture medium or contacting the live virus with the outside of the cell.

Following transfection with the expression vector, the transfected cell is grown under conditions which allow for expression from the hybrid promoter, including in the presence of any transcription factors or cell signals required for transcription from the particular hybrid promoter being used. For example, where the viral enhancer requires a viral protein or a particular cellular protein not typically expressed in glial cells, such a growth factor or protein should be supplied or be expressed in the transfected cell, for example by inclusion of a gene encoding such a factor in the expression but under control of a promoter that does not require such a factor.

Also described is a method for treating a neural-related disorder in a subject. The neural-related disorder in one particular embodiment is a glioma, which may be an astrocytoma. In another particular embodiment the neural-related disorder is a neuronal cancer, which may include a medulloblastoma. In other embodiments the neural-related disorder is epilepsy, a neurodegenerative disorder such as Alzheimer's or Parkinson's, or damage of neurons or astrocytes due to alcohol exposure.

"Treating" refers to refers to an approach for obtaining beneficial or desired results, including clinical results. Beneficial or desired clinical results can include, but are not limited to, alleviation or amelioration of one or more symptoms or conditions, diminishment of extent of disease, stabilization of the state of disease, prevention of development of disease, prevention of spread of disease, delay or slowing of disease progression, delay or slowing of disease onset, amelioration or palliation of the disease state, and remission (whether partial or total). "Treating" can also mean prolonging survival of a patient beyond that expected in the absence of treatment. "Treating" can also mean inhibiting the progression of disease, slowing the progression of disease temporarily, although more preferably, it involves halting the progression of the disease permanently.

The method includes administering to a subject a nucleic acid molecule, for example an expression vector, containing a coding sequence encoding a therapeutic product operably linked to a hybrid promoter comprising a neural-specific promoter and a viral enhancer. The nucleic acid molecule may further include ITRs flanking the operably linked hybrid promoter and coding sequence.

The subject is any subject in need of such treatment, including a mammal, particularly a human subject.

Methods for introducing the nucleic acid molecule into mammalian cells *in vivo* are known, including for gene therapy and may be used to administer the nucleic acid molecule of the invention, such as an expression vector, to a subject for gene therapy of neural-related disorders. A nucleic acid of the invention may be delivered to cells in a subject using methods such as direct injection of DNA, receptor-mediated DNA uptake, viral-mediated transfection or non-viral transfection and lipid based transfection, all of which may involve the use of expression vectors as described herein. A delivery apparatus (e.g., a "gene gun") for injecting DNA into cells *in vivo* may be used. Such an apparatus may be commercially available (e.g., from BioRad).

To deliver the nucleic acid molecule specifically to neural cells such as neurons, or such as glial cells, including astrocyte cells or astrocytoma cells in a particular region of the brain, the nucleic acid molecule, particularly a viral expression vector, may be administered by injection, including stereotaxic microinjection as is known in the art. For human patients, the stereotactic frame base will be fixed into the skull, and the brain with the stereotactic frame base will be imaged using high resolution MRI. Using appropriate stereotactic software, the images will be translated into 3 dimensional coordinates appropriate for the stereotactic frame for targeted injection of vector DNA.

The nucleic acid molecule is administered in an amount sufficient to achieve the desired result, for example, expression of the therapeutic product in the target cells: For example, the nucleic acid molecule may be administered in quantities and dosages necessary to express sufficient quantities of a therapeutic product which functions to alleviate, improve, mitigate, ameliorate, stabilize, inhibit, prevent including prevent the spread of, slow or delay the progression of, or cure a neural-related disease or disorder.

The effective amount to be administered to a patient can vary depending on many factors such as the pharmacodynamic properties of the nucleic acid molecule, the mode of administration, the age, health and weight of the subject, the nature and extent of the disorder or disease state, the frequency of the treatment and the type of concurrent treatment, if any.

One of skill in the art can determine the appropriate amount based on the above factors. The nucleic acid molecule may be administered initially in a suitable amount that may be adjusted as required, depending on the clinical response of the patient. The effective amount of nucleic acid molecule can be determined empirically and depends on the maximal amount of the nucleic acid molecule that can be administered safely.

When the nucleic acid molecule comprises a viral expression vector, the virulence and titre of the virus will be a factor in determining the effective amount.

Particularly, when the nucleic acid molecule is in the form of a baculovirus, since baculovirus has little to no cytotoxicity in vertebrates, large doses may be tolerated. However, the amount of baculovirus administered should be the minimal amount that produces the desired result. A dose of about 10⁹ plaque forming units ("pfu") of baculovirus as described herein may be administered to a human patient. About 10² to about 10⁹ pfu, about 10⁶ to about 10⁹ pfu, about 10⁵ to about 10⁷ pfu, or about 10⁵ to about 10⁶ pfu may be administered in a single dose.

Effective amounts of baculovirus can be given repeatedly, depending upon the effect of the initial treatment regimen. Administrations are typically given periodically, while monitoring any response. It will be recognized by a skilled person that lower or higher dosages than those indicated above may be given, according to the administration schedules and routes selected.

The nucleic acid molecule may be administered as a sole therapy or may be administered in combination with other therapies, including chemotherapy, radiation therapy or other gene therapies. For example, the nucleic acid molecule may be administered either prior to or following surgical removal of a primary tumour or prior to, concurrently with or following treatment such as administration of radiotherapy or conventional chemotherapeutic drugs. The nucleic acid molecule can be administered in combination with, or in a sequential fashion with, an oncolytic virus that induces lysis of a cell that becomes infected with the oncolytic virus.

Also presently contemplated are uses of the various nucleic acid molecule constructs described herein for expressing an expression product in a neural cell or in a cell derived from a neural cell, and for treating a neural-related disorder in a subject, particularly for treating a neuronal cancer or a glioma in a subject. Also contemplated are uses of the various nucleic acid molecule constructs described herein for preparation of a medicament for treating a neural-related disorder in a subject, particularly for treating a neuronal cancer or a glioma in a subject.

Transgenic cells and transgenic non-human animals comprising the present hybrid promoter, expression cassette or expression vector are also contemplated.

To aid in administration, the nucleic acid molecule may be formulated as an ingredient in a pharmaceutical composition. The compositions may routinely contain pharmaceutically acceptable concentrations of salt, buffering agents, preservatives and various compatible carriers or diluents. For all forms of delivery, the nucleic acid molecule may be formulated in a physiological salt solution.

The proportion and identity of the pharmaceutically acceptable diluent is determined by chosen route of administration, compatibility with a nucleic acid molecule, compatibility with a live virus when appropriate, and standard pharmaceutical practice. Generally, the pharmaceutical composition will be formulated with components that will not significantly impair the biological properties of the nucleic acid, particularly when it is a baculovirus expression vector. Suitable vehicles and diluents are described, for example, in Remington's Pharmaceutical Sciences (Remington's Pharmaceutical Sciences, Mack Publishing Company, Easton, Pa., USA 1985).

Solutions of the nucleic acid molecule may be prepared in a physiologically suitable buffer. Under ordinary conditions of storage and use, these preparations contain a preservative to prevent the growth of microorganisms, but that will not inactivate or degrade the nucleic acid molecule. A person skilled in the art would know how to prepare suitable formulations. Conventional procedures and ingredients for the selection and preparation of suitable formulations are described, for example, in Remington's Pharmaceutical Sciences and in The United States Pharmacopeia: The National Formulary (USP 24 NF19) published in 1999.

The forms of the pharmaceutical composition suitable for injectable use include sterile aqueous solutions or dispersion and sterile powders for the extemporaneous preparation of sterile injectable solutions or dispersions, wherein the term sterile does not extend to any live virus that may comprise the nucleic acid molecule that is to be administered. In all cases the form must be sterile and must be fluid to the extent that easy syringability exists.

Kits and commercial packages containing the various nucleic acid molecule constructs described herein, including an expression vector containing a coding sequence encoding a therapeutic product operably linked to a hybrid promoter comprising a neural-specific promoter and a viral enhancer, or kits and commercial packages containing a pharmaceutical composition as described herein, are contemplated. Such a kit or commercial package will also contain instructions regarding use of the included nucleic acid molecule or pharmaceutical composition, for example, use to treat a neural-related disorder, for example, a glial-related disorder such as a glioma, or for expressing an expression product in a neural cell or in a cell derived from a neural cell.

### EXAMPLES

**EXAMPLE 1**

**MATERIALS AND METHODS**

**Construction of a hybrid promoter and recombinant baculovirus vectors**: To generate the hybrid CMV E/GFAP promoter, the CMV enhancer (-568 to -187 relative to the TATA box of the CMV immediate-early promoter) amplified from pRC/CMV₂ (Invitrogen, CA, USA) was inserted into pFastBac1 (Gibco BRL, Life Technologies, Gaithersburg, MD, USA) between the sites of Not I and Xba I, and a GFAP promoter amplified from pDRIVE02-GFAP (InvivoGen, San Diego, CA, USA) was subsequently inserted at the downstream of the CMV E between Xba I and Xho I. Two constructs containing the CMV full-length promoter or the GFAP promoter, respectively, were generated by inserting the promoters into pFastBac1 either between Not I and Xba I (for the CMV promoter) or between Xba I and Xho I (for the GFAP promoter). A luciferase cDNA from pGL3-basic vector (Promega, Madison, WI, USA) was used as the reporter gene for all the constructs and inserted into the downstream of the promoters, between the sites of Xho I and Hind III. In some experiments, the p53 tumor suppressor gene was used and inserted downstream of the hybrid promoter between the sites of Xho I and Hind III. To construct a baculovirus vector with AAV ITRs, an expression cassette from pAAV-MCS-luc (Wang et al., J Gene Med. (2005) Jul;7(7):945-55) containing a multiple cloning site (MCS), a reporter gene encoding luciferase, a SV40 polyA signal, and two ITR sequences at two ends was amplified and inserted into pFastBac1 between Avr II and Sal I. The hybrid CMV E/GFAP promoter was then inserted into the sites of Kpn I and Hind III.

Recombinant baculovirus vectors with the luciferase gene under the control of the full-length CMV promoter, the GFAP promoter, the hybrid CMV E/GFAP promoter and the hybrid CMV E/GFAP promoter plus AAV ITRs were produced and propagated in Sf9 insect cells according to the manual of the Bac-to-Bac baculovirus expression system (Gibco BRL, Life Technologies, Gaithersburg, MD, USA), and named BV-CMV, BV-GFAP, BV-CMV E/GFAP and BV-CMV E/GFAP-ITR, respectively. The baculovirus vector containing p53 was named BV-CMV E/GFAP-p53. Budded viruses in the insect cell culture medium were collected and filtered through a 0.4-µm pore size filter (Minipore, Bedford, MA, USA) to remove any contamination, and concentrated by ultracentrifugation at 25,000g for 60 min. Viral pellets were resuspended in appropriate volumes of 0.1 M phosphate-buffered saline (PBS) and their infectious titers (plaque-forming units, pfu) were determined by plaque assay on Sf9 cells.

**Virus infection**: For *in vitro* infection, primary glial cell cultures established from the cortices of embryonic Wistar rats at gestational day 20, and glioma cell lines including C6, H4, SW1088, SW1783, U87, BT325 and U251 were used. Cells were seeded in wells of 48-well plates at density of 20,000 cells per well and infected with appropriate amounts of baculovirus vectors in 100 µl of serum free DMEM at 37°C for 1 h. After the incubation, serum free DMEM containing the viruses was replaced by normal growth medium and the cells were further cultured at 37°C for indicated periods of time.

For *in vivo* viral delivery, adult male Wistar rats (weighing 250-300 g) were used. Five microliters of baculovirus vectors was injected stereotaxically into the striatum at two injection sites (AP ±1.0mm, ML +2.5mm, and DV -5.0mm from bregma and dura), using a 10 µl Hamilton syringe connected with a 30-gauge needle at a speed of 0.5 µl/min. The needle was allowed to remain in place for another 5 min before being slowly retracted at the end of each injection. Rats were sacrificed and the brain tissues were collected at indicated time points after the injection.

**Luciferase assay**: To prepare supernatants for luciferase activity assays, transduced cultured cells were washed and permeabilized with 100 µl of reporter cell lysis buffer (Promega) and brain tissue samples were homogenized in PBS (100 µl PBS per 50 mg tissue) by sonication for 10 s on ice and centrifuged at 13 000 rpm for 10 min at 4°C. Ten µl of the cell extract or the supernatant of homogenized tissues was used for luciferase assay with an assay kit from Promega. Measurements were made in a single-tube luminometer (Berthold Lumat LB 9507, Bad Wildbad, Germany) for 10 seconds. The results were expressed in relative light units (RLU) per milligram of total protein or per brain region.

In an experiment measuring luciferase activity in the rat cerebral cortex after striatum injection of viral vectors, the tissue was dissected into two parts: one around the needle track and the rest. As in a pilot test we observed the leakage of viral vectors into the cerebral cortex during needle penetration or withdraw, the pieces of the cerebral cortex tissues around the needle track were not included in the analysis in order to exclude false readings in the region caused by the leaked viral vectors.

**Immunohistochemical analysis**: After receiving deep anesthesia, rats were perfused with 0.1 M PBS (pH 7.4) solution followed by 4% paraformaldehyde in PBS. The brains were removed and postfixed in the same fixative for 2-4 h before being transferred into 20% sucrose in 0.1 M PBS to incubate overnight at 4°C. Cryostat sections, within 0.5 mm from the injection track, were cut at 30 µm thickness for free-floating immune staining. A polyclonal anti-luciferase (Promega, dilution 1:150), a monoclonal anti-GFAP protein (Chemicon International, USA; dilution 1:150) and a monoclonal anti-neuron-specific nuclear protein (NeuN) (Chemicon, dilution 1:500) were used as primary antibodies. Anti-rabbit IgG TRITC conjugate (Sigma-Aldrich, USA; dilution 1:500) and anti-mouse IgG FITC conjugate (Sigma-Aldrich; dilution 1:500) were used as the secondary antibodies. Sections were examined with an Olympus Fluoview 300 confocal laser scanning microscope. Each section was initially scanned with a 488 nm laser line, and an emission filter BP 510-530 for the detection of FITC fluorescein, then with a 543 nm laser line and an emission filter LP 560 for the detection of TRITC.

**MTT assay**: Cells were seeded in 96-well plates at density of 10,000 cells per well one day before viral infection. The cells were infected with appropriate amounts of recombinant baculoviruses in 50 µl of serum free DMEM and incubated at 37°C for 1 h. After the incubation, serum free DMEM containing the baculoviruses was replaced by 100 µl of fresh growth medium, and the cells were continued to incubate at 37°C for 5 days. At day 5, 10 µl of MTT solution (5 mg/ml in PBS, sterilized by filtration) was added to each well and after 4 h incubation at 37°C the reaction was stopped by adding 100 µl of 10% SDS in 0.01 M HCl. Following further overnight incubation of the plates at 37°C, the absorbance at 595nm was measured in a microplate reader (BioRad, Module 550).

**Rat C6 brain tumor model and tumor growth monitoring**: Adult male Wistar rats (weighing 250-320 g) were anesthetized by injection (i.p.) of sodium pentobarbital (60 mg/kg b.w.). The rats were positioned in a stereotaxic instrument with the nose bar set at 0. Rat glioma C6 cells stably transfected with a luciferase reporter gene (C6-luc) were used for inoculation. Five µl (1 x 10⁵) of C6-luc cells in PBS was injected into the striatum, with or without BV-CMV E/GFAP-p53 at MOI of 100, respectively. The coordinates of injections were: AP ±1.0mm, ML +2.5mm, and DV -5.0mm from bregma and dura. The injections were done using a 10 µl Hamilton syringe at a speed of 0.5 µl/min and the needle was allowed to remain in place for another 5 min before being slowly retracted at the end of each injection.

The tumor growth was monitored by either luminescent imaging of C6-luc cells in living animals or luciferase activity assays of brain tissues. Luminescent imaging was performed with an IVIS Imaging System (Xenogen, Alameda, California, USA) comprised of a highly sensitive, cooled CCD camera mounted in a light-tight specimen box. Ten minutes prior to *in vivo* imaging, anesthetized animals inoculated with C6-luc were intraperitoneally injected D-luciferin (Biosynth, Naperville, Illinois, USA) at 150 mg/kg in PBS. The animals were then placed onto a warmed stage inside the camera box. The light emitted from C6-luc cells was detected *in vivo* by the IVIS Imaging System and digitized and electronically displayed as a pseudocolor overlay onto a gray scale animal image. Images and measurements of luminescent signals were acquired and analyzed using the Living Image software (Xenogen). For luciferase activity assays, tissue samples from two sides of the brain were collected and processed as stated above.

**DETAILED FIGURE LEGENDS**

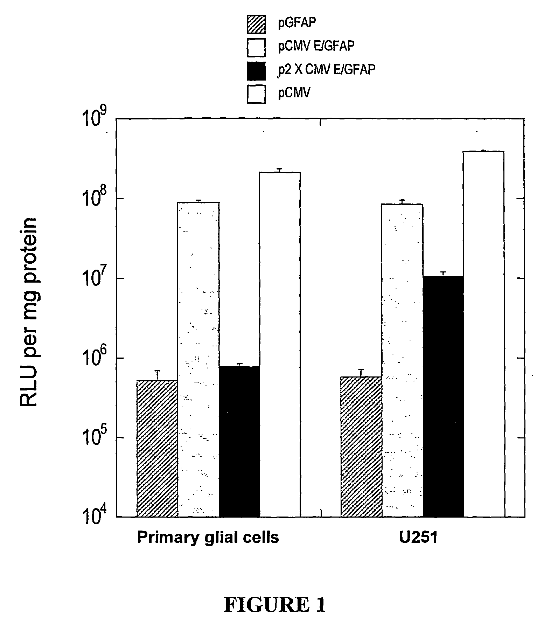

**Figure 1****.** Effects of the CMV enhancer on the activity of a GFAP promoter in the context of plasmid vectors. Primary glial cells from the rat cortex and human U251 astrocytoma cells were transfected with four constructs with different gene regulatory elements, each of which bears a downstream luciferase reporter gene. Luciferase activity assays were performed in quadruplicate and the standard deviation is indicated with error bars. The results are presented in relative light units (RLU) per milligram of total cell protein.

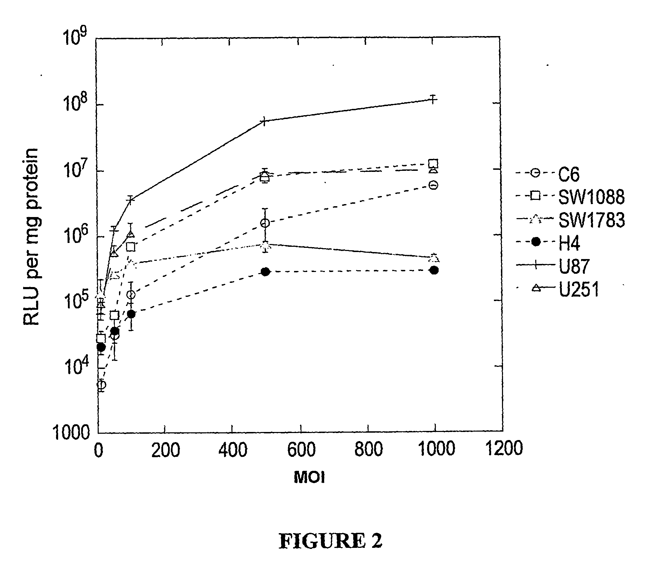

**Figure 2****.** Dose-dependent gene expression of luciferase reporter gene from BV-CMV in glioma cells. Glioma cells were infected at different multiplicities of infection (MOI) from 10 to 1000. Luciferase activity assays were performed 2 days after in quadruplicate and the standard deviation is indicated with error bars. The results are presented in relative light units (RLU) per milligram of total cell protein.

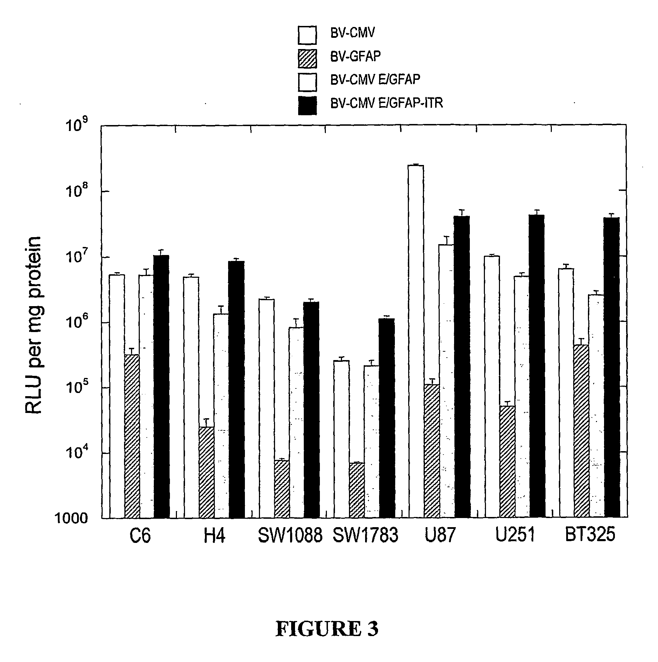

**Figure 3****.** Activities of different regulatory elements in the context of baculovirus vectors in glioma cells. Cells were infected with four types of baculovirus vectors with different gene regulatory elements at the same multiplicity of infection (MOI = 25). Luciferase activity assays were performed 2 days after in quadruplicate and the standard deviation is indicated with error bars. The results are presented in relative light units (RLU) per milligram of total cell protein.

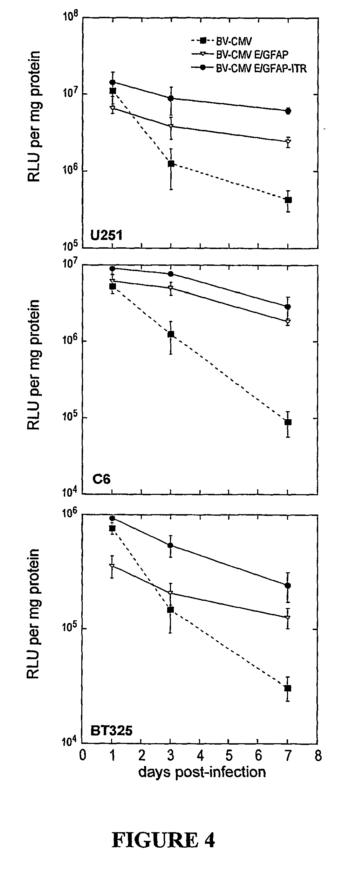

**Figure 4**. Kinetics of luciferase expression in glioma cell lines infected with recombinant baculoviruses. Three glioma cell lines (U251, C6 and BT325) were infected with BV-CMV, BV-CMV E/GFAP or BV-CMV E/GFAP-ITR at the same multiplicity of infection (MOI = 25). Luciferase activity assays were performed in quadruplicate 1, 3 and 7 days after infection and the standard deviation is indicated with error bars. The results are presented in relative light units (RLU) per milligram of total cell protein.

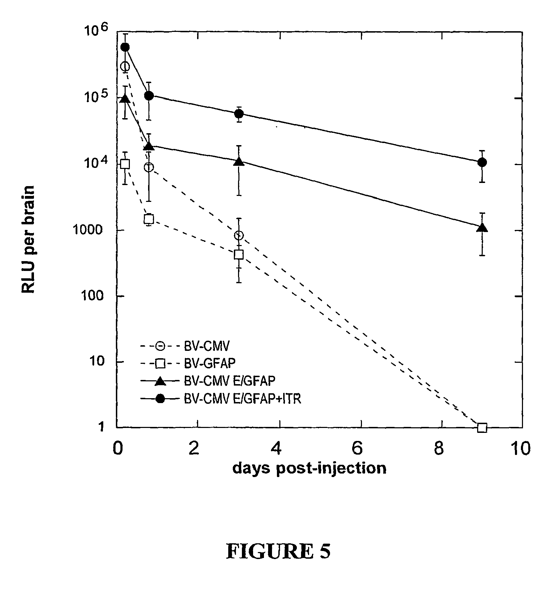

**Figure 5****.** Luciferase gene expression in rat brains after single injection of recombinant baculoviruses. Five µl (5 x 10⁶ pfu) of BV-CMV, BV-GFAP, BV-CMV E/GFAP, or BV-CMV E/GFAP-ITR was injected into the rat striatum. Four rats per group were used at each time point. Values are expressed as RLU per brain and the standard deviation is indicated with error bars.

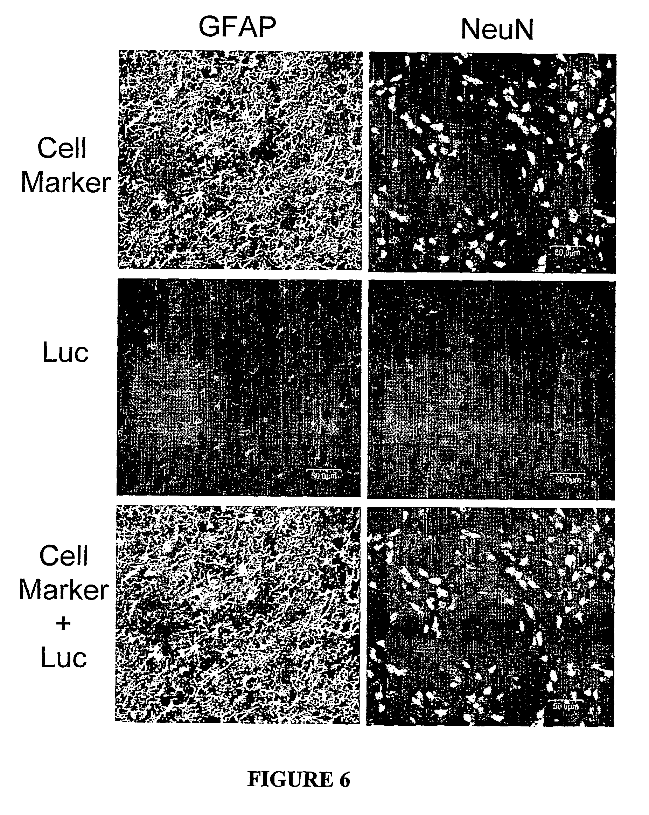

**Figure 6****.** Immunohistological analysis of rat brains injected with recombinant baculoviruses. Five µl (1 × 10⁸ pfu) of BV-CMV E/GFAP-ITR was injected into the striatum. Two days after injection, brain samples were collected and frozen coronal sections of each brain, within 0.5 mm from the injection site, were cut at 30 µm thickness for free-floating immunostaining.

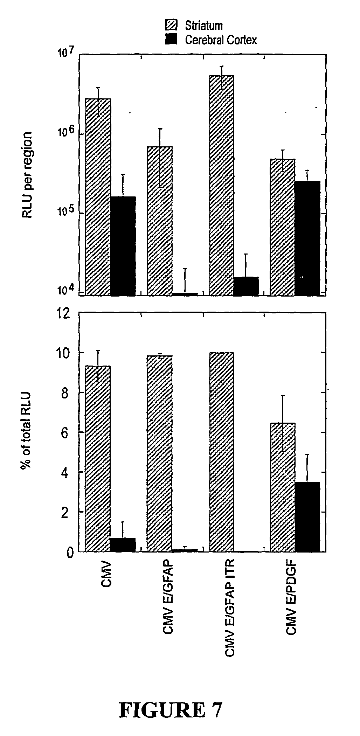

**Figure 7****.** GFAP promoter activity in neurons as measured by luciferase expression in a brain region remote from an injection site. Two days after injection of viral vectors with different gene regulatory elements (1 × 10⁸ pfu), rat brain tissues from the striatum (the injection site) and the cerebral cortex (the remote region) were collected. Their luciferase activities were measured and expressed as RLU per region (means ± s.d., n=4) and as % of total RLU.

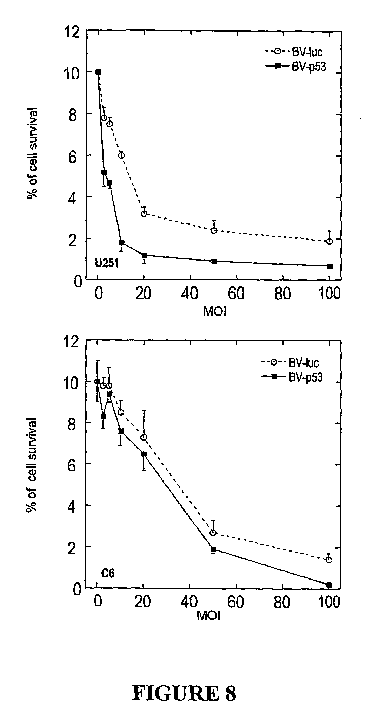

**Figure 8****.** Viabilities of cells infected with baculoviruses with the wild-type p53 gene or a luciferase reporter gene. U251 or C6 glioma cells were infected with BV-CMV E/GFAP-p53 or BV-CMV E/GFAP-luc at a MOI of 0, 25, 50, 100, 200, 500 or 1000. The viabilities of the cells were measured by MTT assay 5 days after the infection. All values were normalized to the relative viability of the mock-infected controls. The results represent the average obtained from four independent experiments with error bars representing the standard deviation.

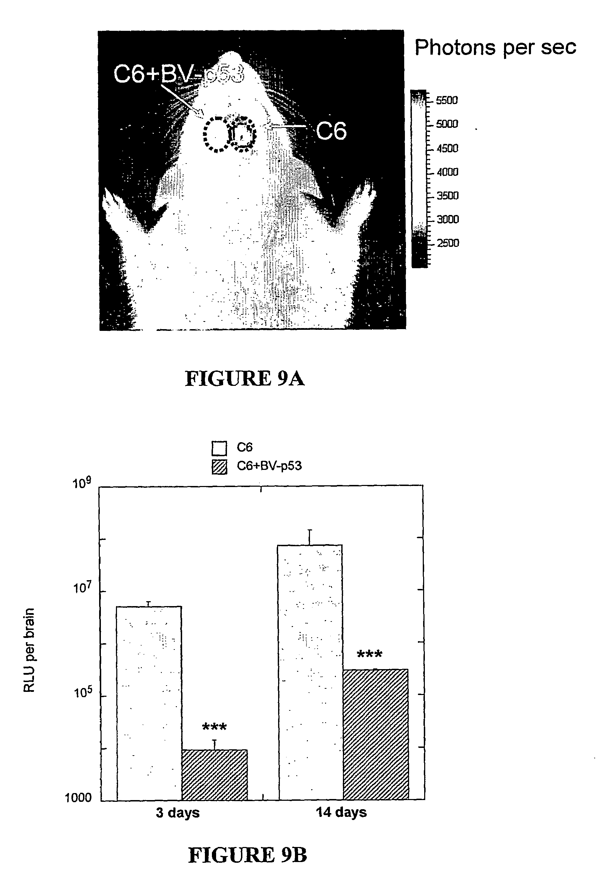

**Figure 9****.** In vivo assays of C6 tumor growth in the rat brain. Anesthetized rats were inoculated with 1 x 10⁵ C6-luc cells to each side of the brain, with or without BV-CMV E/GFAP-p53 ("BV-p53") at a MOI of 100, respectively. A: In vivo monitoring of the light emitted from C6-luc cells inoculated 14 days earlier in a living animal. B: Luciferase activity assays of brain tissues collected 3 and 14 days after inoculation. *** P<0.001 compared with the animals inoculated with C6 without BV-p53.

**RESULTS**

**Improved transgene expression in cultured cells**: To test whether the CMV enhancer would be able to improve the strength of a GFAP promoter, we first constructed and examined plasmid vectors. Gene regulatory elements from pDRIVE02-GFAP(r) v04 containing a rat GFAP promoter (InvivoGen, San Diego, CA, USA) and pRc/CMV2 containing enhancer-promoter sequences from the immediate early gene of the human CMV (Invitrogen, Carlsbad, CA, USA) were cloned into the same type of luciferase reporter vector, pGL3-basic vector (Promega, WI, USA). Four types of plasmid vectors were constructed to contain the GFAP promoter (pGFAP), a hybrid promoter with the CMV enhancer inserted upstream of the GFAP promoter (pCMV E/GFAP), a hybrid promoter with two copies of the CMV enhancer placed upstream of the GFAP promoter (p2 x CMV E/GFAP) or the CMV immediate-early enhancer/promoter (pCMV). Luciferase expression levels from the four plasmid vectors were compared following the transfection of primary rat glial cells and U251 human astrocytoma cells with plasmid DNA complexed with polycation polyethylenimine (PEI). As shown in Figure 1, pCMV E/GFAP significantly improved gene expression when compared with pGFAP, by about 100-fold in the two types of cells tested. The expression levels from the hybrid promoter were just slightly lower than those of the CMV enhancer/promoter, which is considered as one of the strongest transcriptional control elements. However, levels of expression were notably reduced by the addition of a second copy of the CMV enhancer in both primary glial cells and U251 cells.

We then constructed recombinant baculovirus vectors by inserting the reporter gene encoding luciferase (luc) under the control of the CMV, GFAP, or CMV E/GFAP promoter into the transfer vector pFastBac1 (Invitrogen, USA) and named here BV-CMV, BV-GFAP, and BV-CMV E/GFAP, respectively. A fourth expression cassette was constructed by flanking CMV E/GFAP with AAV ITR sequences and the generated chimeric AAV/baculovirus vector was named BV-CMV E/GFAP-ITR. In view of the broad activity of the CMV promoter in various types of cells, we started our virus experiments by using BV-CMV to test baculovirus infection efficiency in 6 glioma cell lines, namely C6, H4, SW1088, SW1783, U87, and U251. In general, a dose-dependent response was observed in all glioma cells tested, with U87 displaying a steepest slope (Fig. 2).

The next experiment was to compare gene expression levels of the above recombinant baculovirus with different expression cassettes in glioma cells using a quantitative luciferase activity assay. Similar to that observed by using plasmid vectors, the CMV enhancer in the context of baculovirus could significantly increase the strength of the GFAP promoter by 10 to 100-fold, dependent on the cells tested, resulting in levels of gene expression close to those of the CMV full-length promoter (Fig. 3). With AAV ITR flanking, further improvement in gene expression was obvious. When compared with BV-CMV E/GFAP, BV-CMV E/GFAP-ITR increased the expression at least 10-fold in C6, U87, BT325 and U251 cells, reaching the levels significantly higher than those provided BV-CMV (Fig 3).

To test whether the CMV enhancer and the AAV ITR sequences could prolong transgene expression, we analyzed kinetics of gene expression in cultured C6, BT325 and U251 cells in a 7-day experiment. As shown in Figure 4, the levels of gene expression from BV-CMV dropped much faster than those from BV-CMV E/GFAP and BV-CMV E/GFAP-ITR. BV-CMV resulted in higher levels of gene expression compared to BV-CMV E/GFAP at the beginning in two of the three cell lines, which however became much lower than those of BV-CMV E/GFAP by day 7. Among three viral vectors tested, BV-CMV E/GFAP-ITR was the best, mediating the highest levels of gene expression in all three cell lines at all time points examined.

**Improved transgene expression in the brain**: The possibility of using new baculovirus vectors to enhance and extend gene expression was further investigated in the rat brain after stereotaxical injection of viral vectors (5×10⁶ pfu per animal) into the striatum, followed by analysis of luciferase expression at different time points up to 90 days (Fig. 5). BV-CMV mediated an initially high but sharply dropping luciferase expression, which was undetectable after 30 days. Similar to that observed *in vitro*, BV-GFAP performed poorly as well in the brain, mediating a low level and short duration of gene expression. BV-CMV E/GFAP -mediated luciferase expression also decreased over time but at a much slower rate, showing higher levels than those from BV-CMV from day 3 onwards. With ITR flanking of the expression cassette, further improvement was observed, resulting in luciferase expression levels 10 times higher than BV-CMV E/GFAP. The superior performance of BV-CMV E/GFAP-LTR over BV-CMV E/GFAP was maintained through the whole experimental period, although the luciferase expression level decreased time-dependently too.

**Astrocyte-specific expression in the brain**: One of the critical issues in developing a hybrid promoter is whether the original cellular specificity of a cell-type promoter could be preserved after promoter engineering. We examined the issue with two approaches. Firstly, immunohistochemical double-staining was carried out. Two days after the injection of 5 µl (1×10⁸ pfu) of either BV-CMV E/GFAP or BV-CMV E/GFAP-ITR into the rat striatum, brain tissue sections were collected for double-immunostaining using anti-luciferase antibodies to visualize transducted cells, anti-GFAP antibodies or anti-neuronal nuclei protein (NeuN) antibodies to show astrocytes or neurons, respectively. The luciferase-GFAP double staining of tissue sections collected from the regions around the injection site of BV-CMV E/GFAP injected animals demonstrated that luciferase expression was almost exclusively in astrocytes. Among total of 564 cells stained positively with the anti-luciferase antibody, 525 of them (93%) were simultaneously stained with the GFAP antibody. In the case of using BV-CMV E/GFAP-ITR, almost all transduced cells in the striatum were also GFAP-positive (Fig. 6). As low endogenous levels of GFAP could lead to false negative staining, the luciferase-NeuN double staining was carried out to examine whether gene expression occurred in neurons. Consistent with the finding from the luciferase-GFAP double staining, none of the luciferase-positive cells were positively stained using anti-NeuN antibodies (Fig. 6).

Our previous study demonstrated that baculoviruses, after being internalized by nerve terminals at an injection site, could migrate by axonal transport to neuronal cell bodies in a remote region (Li et al., (2004), *supra*). This feature offers a unique opportunity to study specificity and activity of a promoter in neurons, as gene expression detected in a region remote from the injection site would result from axonal transport of baculovirus vectors and be a pure neuronal expression. This viral transport mechanism was used in the current study to examine the cellular specificity of newly developed vectors containing the GFAP promoter by analyzing gene expression in the striatum, the injection site, and in the cerebral cortex, a remote region where some of neurons have long axons projecting to the striatum (Fig. 7). The luciferase expression from BV-CMV was detected mainly at the injection site, with 93% of total expression in the striatum and 7% in the cerebral cortex, indicating certain activity of the CMV promoter in neurons. A baculovirus vector with a neuron-specific promoter (Li et al., (2004), *supra*), included here as a positive control, provided a high level of luciferase expression, around 35% of total value, in the remote cerebral cortex region. When two baculovirus vectors containing the GFAP promoter were tested, extremely low expression was detected in the cerebral cortex, 1% from BV-CMV E/GFAP and only 0.3% from BV-CMV E/GFAP-ITR, suggesting that the two baculovirus vectors containing the GFAP promoter did not display or display very low activity in neurons.

**Growth inhibition of glioma cells**: Having shown improved gene expression from our baculovirus vector, we explored the application of the viral vector for cancer gene therapy in the central nervous system. The tumor suppressor p53 gene was cloned into the baculovirus vector downstream of the hybrid CMV enhancer/GFAP promoter and the produced vector was named BV-CMV E/GFAP-p53. The vector was first tested in glioma cell lines *in vitro.* U251 and C6 cells were infected with BV-CMV E/GFAP-p53 at a range of ascending multiplicities of infection (MOI) of 0, 25, 50, 100, 200, 500 and 1000, and the viability of cells was measured by MTT assay 5 days after the infection. The cells infected with a baculovirus vector with the luciferase gene were used as control. The results demonstrate glioma cell death in a dose-dependent manner in U251 cells infected with baculovirus vectors with the p53 gene, with almost 90% of glioma cell death at a MOI of 200 (Fig 8). A dose-dependent cell death was also observed in U251 cells infected with the control baculovirus vectors with the luciferase gene, albeit slightly less pronounced, resulting in death of 80% of cells at a MOI of 1000. Similar effects of the control baculovirus vectors on cell survival were observed also in C6 cells, which did not differ significantly from what caused by BV-CMV E/GFAP-p53 at most of viral concentrations tested. However, at a MOI of 1000, all the C6 glioma cells were killed by BV-CMV E/GFAP-p53 while around 20% of the cells infected by the control vector were still alive (Fig. 8).

An *in vivo* study was performed by injection of C6-luc cells, or the cells together with BV-CMV E/GFAP-p53, into the rat striatum. The C6 cells were stably transfected with a luciferase gene in advance to facilitate quantitative evaluation of tumour growth. Tumour cell growth in the brain was assayed 3 and 14 days post-injection by luminescent imaging in living animals or by measuring luciferase activity of the brain samples. Figure 9A shows the easily detected light from C6-luc cells inoculated into the brain without baculoviruses and an obvious inhibition of BV-CMV E/GFAP-p53 on C6-luc cell growth in the brain of a living rat 14 days after the inoculation. Quantitative analysis of luciferase activities of brain tissue samples demonstrated significant differences in tumor growth rate between the two groups of animals, with significantly higher levels of luciferase activities in the C6-luc group than those in the group injected with BV-CMV E/GFAP-p53 together with C6-luc cells at day 3 and 14, respectively (Fig. 9B).

**EXAMPLE 2**

**MATERIAL AND METHODS**

**Recombinant baculovirus vectors**: Seven recombinant baculovirus vectors with different expression cassettes were constructed using the transfer vector pFastBac1 (Invitrogen, CA, USA, Table 1). Two of them contain the CMV enhancer/promoter to drive expression of a firefly luciferase reporter gene (BV-CMV-Luc) and an EGFP reporter gene (BV-CMV-EGFP), respectively. Three baculovirus vectors carry the GFAP promoter to drive expression of the luciferase gene, the first of which has an unmodified GFAP promoter (BV-GFAP-Luc), the second, a modified GFAP promoter produced by appending the CMV enhancer (-568 to -187 relative to the TATA box) upstream to the GFAP promoter (BV-CMV E/GFAP-Luc) and the third, an expression cassette produced by flanking the second cassette with AAV ITRs (BV-CG/ITR-Luc). The other two vectors were produced by replacing the luciferase gene in BV-CG/ITR-Luc with a DT-A gene or an EGFP gene and named BV-CG/ITR-DTA and BV-CG/ITR-EGFP, respectively.

To generate BV-CMV E/GFAP-Luc, a CMV enhancer sequence amplified from pRC/CMV₂ (Invitrogen) was inserted into pFastBac1 between the sites of *Not* I and *Xba* I, and a GFAP promoter amplified from pDRIVE02-GFAP (InvivoGen, San Diego, CA, USA) was subsequently inserted downstream of the CMV E between *Xba* I and *Xho* I. To construct BV-CG/ITR-Luc, BV-CG/ITR-DTA and BV-CG/ITR-EGFP, an expression cassette from pAAV plasmid (15), containing a multiple cloning site (MCS), a reporter gene encoding luciferase, a SV40 polyA signal, and two ITR sequences at both ends, was amplified and inserted into pFastBac1 between Avr II and *Sal* I. The CMV E/GFAP promoter was then inserted into the sites of *Kpn* I and *Hind* III. A luciferase cDNA from pGL3-basic vector (Promega, Madison, WI, USA), an EGFP reporter gene from pEGFP-C1 vector (Clontech, Mountain View, CA, USA), and a DT-A gene amplified from pCAG/DT-A-2 (kindly provided by Dr Masahiro Sato, Tokai University, Japan) were inserted, respectively, into the downstream of the GFAP promoter between the sites of *Hind* III and *Xba* I. Recombinant baculoviruses were produced and propagated in *Sf9* insect cells according to the manual of the Bac-to-Bac baculovirus expression system (Invitrogen).

**Table 1: Vectors used in Example 2**

| Name | Promoter | Transgene |
|---|---|---|
| BV-CMV-Luc | CMV | Luciferase |
| BV-CMV-EGFP | CMV | EGFP |
| BV-GFAP-Luc | GFAP | Luciferase |
| BV-CMV E/GFAP-Luc | CMV E+GFAP | Luciferase |
| BV-CG/ITR-Luc | CMV E+GFAP, ITR flanking | Luciferase |
| BV-CG/ITR-EGFP | CMV E+GFAP, ITR flanking | EGFP |
| BV-CG/ITR-DTA | CMV E+GFAP, ITR flanking | DT-A |

**Virus infection**: For *in vitro* experiments, human glioma cell lines of BT325, U251, U87, H4, SW1783, and SW1088, rat C6 glioma cell line and two non-glioma cell lines, HepG2 and NIH3T3, were used. A stable C6 cell clone with the firefly luciferase gene under the control of the CMV promoter (C6-Luc) was generated to facilitate bioluminescent imaging in living cells. Cells were seeded in 96-well plates at a density of 1,000 cell per well or 48-well plates at a density of 20,000 cells per well for luciferase activity assay, and in 12-well plates with a density of 100,000 cells per well for flow cytometry analysis. Cells were incubated with appropriate amounts of baculovirus vectors in serum-free DMEM at 37°C for 1 hr. After the incubation, serum-free DMEM containing the viruses was replaced by normal growth medium and the infected cells were further cultured at 37°C until used for various assays.

For *in vivo* experiments, adult male Wistar rats (weighing 250-300 g) were used. Rats were anesthetized and positioned in a stereotaxic instrument with the nose bar set at 0. Rats were first inoculated with rat C6 or C6-luc cells (1 x 10⁵ in 5 µl) in the striatum on one side of the brain (AP+1.0 mm, ML +2.5 mm, and DV -5.0 mm from bregma and dura) using a 10 µl Hamilton syringe connected with a 30-gauge needle at a speed of 0.5 µl/min. The needle was allowed to remain in place for another 5 min before being slowly retracted at the end of each injection. Three days later, 5 x 10⁷ viral particles of BV-CG/ITR-EGFP or 5 x 10⁶ of BV-CG/ITR-Luc in 3 µl were injected into the same region, as well as the contralateral striatum in some animals. Rats were euthanised 2 days after viral injection and the brain tissues were collected for gene expression analysis.

**Transgene expression analysis**: For luciferase activity assays, cultured cells were washed and permeabilized with 100 µl of reporter cell lysis buffer (Promega). Ten µl of the cell extract was used for luciferase assays with an assay kit from Promega. Measurements were made in a single-tube luminometer (Berthold Lumat LB 9507, Bad Wildbad, Germany) for 10 sec. To measure the luciferase expression in brain tissues, samples were collected and homogenized in PBS (100 µl PBS per 50 mg tissue) by sonication for 10 sec on ice and centrifuged at 13 000 rpm for 10 min at 4°C. Ten µl of the supernatant of homogenized tissues was used for luciferase assays.

Luciferase activity in a stable C6 cell clone with the firefly luciferase gene was monitored by luminescent imaging with the IVIS^{®} Imaging System (Xenogen, Alameda, California, USA) comprised of a highly sensitive, cooled CCD camera mounted in a light-tight specimen box. Two to five minutes prior to cell imaging, luciferin-EF (Promega, Madison, WI, USA) at 150 µg/ml in PBS was added to each well. Bioluminescence emitted from the cells was acquired for 30s and quantified as photons/second using the Living Image software (Xenogen).

For flow cytometric analysis for EGFP expression, transduced cells were washed with PBS, trypsinized, dispersed in suspension, and subjected to analyses by FACS Calibur Flow Cytometer (Becton Dickinson, NJ, USA). Untransduced cells served as negative controls. Three sets of independent transduction experiments were carried out for each assay.

For immunohistochemical analysis of cell-type specificity of transgene expression, anesthetized rats were perfused with 0.1 M PBS (pH 7.4) solution followed by 4% paraformaldehyde in PBS. The brains were removed and postfixed in the same fixative for 2-4 hr before being transferred into 20% sucrose in 0.1 M PBS to incubate overnight at 4°C. Cryostat sections were cut at 30 µm thickness for immune staining. A polyclonal anti-luciferase (Promega, dilution 1:150) or a polyclonal anti-GFAP (Promega, dilution 1:150) was used as the primary antibody to show inoculated C6 glioma cells, as well as nearby astrocytes, while the expression of EGFP could be visually detected without staining. Anti-rabbit IgG TRITC conjugate (Sigma-Aldrich, USA; dilution 1:500) was used as the secondary antibody. Mounted sections were examined using a laser scanning confocal microscope.

**MTT assay**: Cells were seeded in 96-well plates at density of 10,000 cells one day before virus infection. The cells were infected with appropriate amounts of recombinant baculoviruses containing either the DT-A gene (BV-CG/ITR-DTA) or EGFP reporter gene (BV-CG/ITR-EGFP) in 50 µl of serum free DMEM and incubated at 37°C for 3 hr. After the incubation, the serum free DMEM containing the baculoviruses was replaced by 100 µl of fresh growth medium, and the incubation of cells was continued at 37°C. At the indicated time points, 20 µl of MTT solution (5 mg/ml in PBS, sterilized by filtration) was added to each well. After 4 hr incubation at 37°C, the medium was removed and 200 µl of DMSO was added into each well to dissolve the crystals. The absorbance was measured in a microplate reader at 550 nm (BioRad, Module 550).

**Rat C6 tumor xenograft model and tumor growth monitoring:** C6-luc cells were injected into the striatum on both sides of the rat brain, 100,000 cells per side, using the protocol described above. Three days later, 1x 10⁷ viral particles in 3 µl per side were injected into the striatum. BV-CG/ITR-DTA was injected into the left side and BV-CG/ITR-EGFP, serving as a viral vector control, into the right side. Tumor growth was monitored by either luminescent imaging of C6-luc cells in living animals or luciferase activity assays of brain tissues. Luminescent imaging was performed with the IVIS Imaging System (Xenogen). Ten minutes prior to *in vivo* imaging, anesthetized animals were intraperitoneally injected with D-luciferin (Promega, WI, USA) at 40 mg/kg in PBS. The animals were then placed onto a warmed stage inside the camera box. The detected light emitted from C6-luc cells was digitized and electronically displayed as a pseudocolor overlay onto a gray scale animal image. Images and measurements of luminescent signals were acquired and analyzed using the Living Image software (Xenogen). Animals were euthanised 14 days after virus injection. For luciferase activity assays, tissue samples from two sides of the brain were collected and processed as stated above.

In the handling and care of animals, *The Guidelines on the Care and Use of Animals for Scientific Purposes* issued by National Advisory Committee for Laboratory Animal Research, Singapore, was followed. For any study that defines death of the experimental animals as the endpoint, the guideline requests consideration of an earlier point in the study when the necessary data have been collected and the animals could be euthanised. The experimental protocols of the current study were approved by the Institutional Animal Care and Use Committee (IACUC), National University of Singapore and Biological Resource Center, the Agency for Science, Technology and Research (A* STAR), Singapore.

**DETAILED FIGURE LEGENDS**

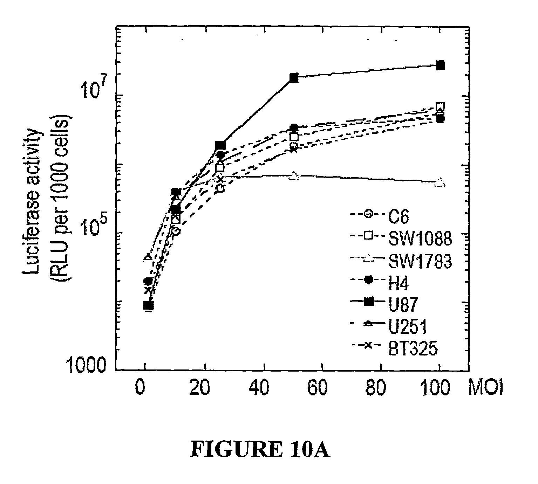

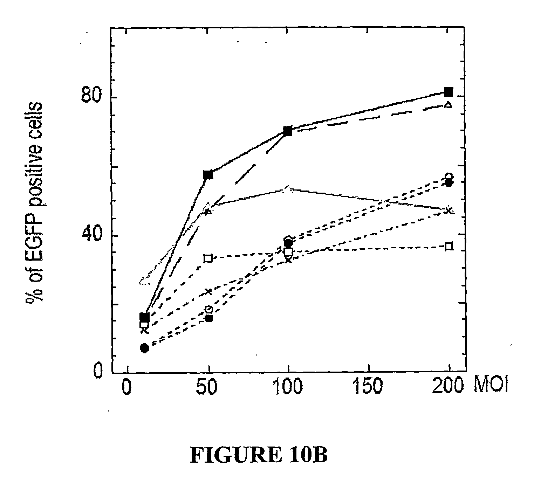

**Figure 10**. Baculovirus-mediated transduction in glioma cells. A: Luciferase expression in glioma cells. Cells were transduced with BV-CMV-Luc with increased MOI from 1 to 100. Luciferase assay was carried out one day after infection. The results are expressed in relative light units (RLU) per 1000 cells. B: EGFP expression in glioma cells. Cells were transduced with BV-CMV-EGFP with increased MOI from 10 to 200 and analyzed with flow cytometry one day later. The results are reported as the percentage of EGFP-positive cells.

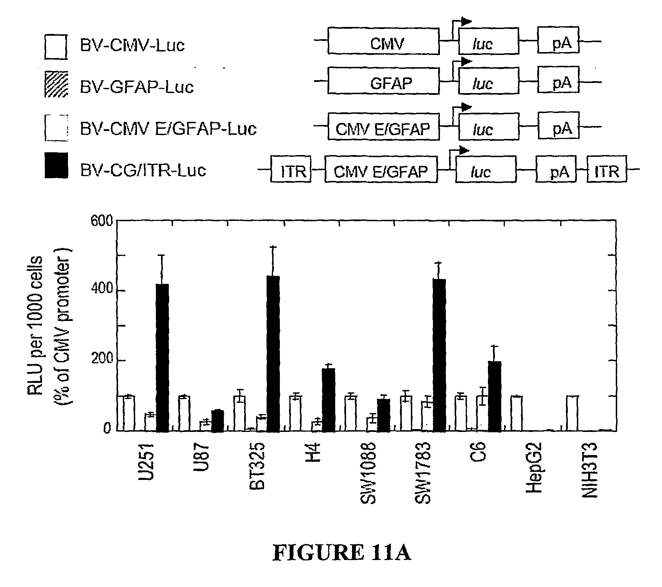

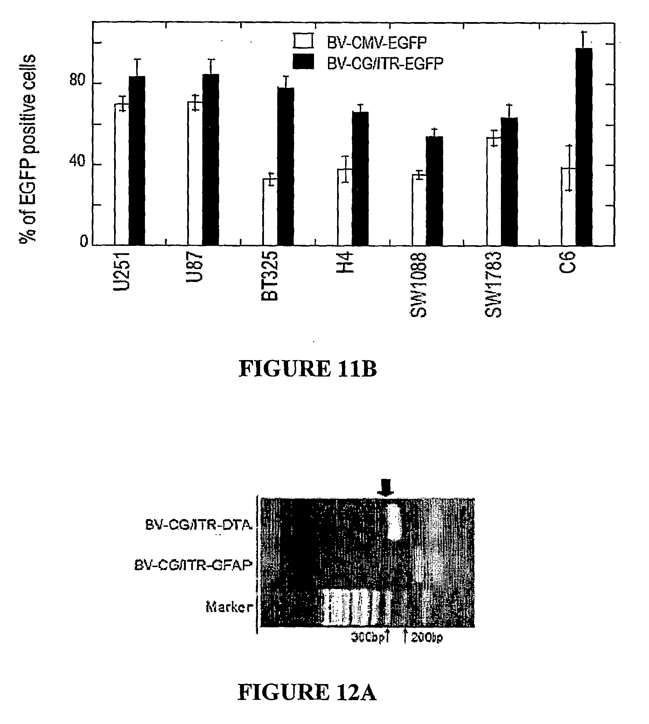

**Figure 11**. Modified GFAP promoters improved baculovirus-mediated transduction in glioma cells. A: Baculoviral vectors with a luciferase reporter gene. Schematic diagram of the expression cassettes is shown on the top. BV, baculovirus; CMV, the promoter/enhancer of human cytomegalovirus immediate-early gene; GFAP, the promoter of the glial fibrillary acidic protein; CMV E, the enhancer of human cytomegalovirus immediate-early gene; ITR, AAV inverted terminal repeats; *luc,* luciferase gene; pA, SV40 polyA signal. Cells were infected with the baculoviral vectors at an MOI of 25. Luciferase activity assay was performed one day after transduction. Results are expressed in relative light units (RLU) per 1000 cells and reported as the percentage of RLUs produced by the vector with the CMV promoter (mean ± SD, n=4). B: Baculoviral vectors with an EGFP reporter gene. Cells were transduced with BV-CG/ITR-EGFP at an MOI of 100 and analyzed with flow cytometry one day later. The results are reported as the percentage of EGFP-positive cells. The results from the experiment with BV-CMV-EGFP in Fig 1B are included for comparison.

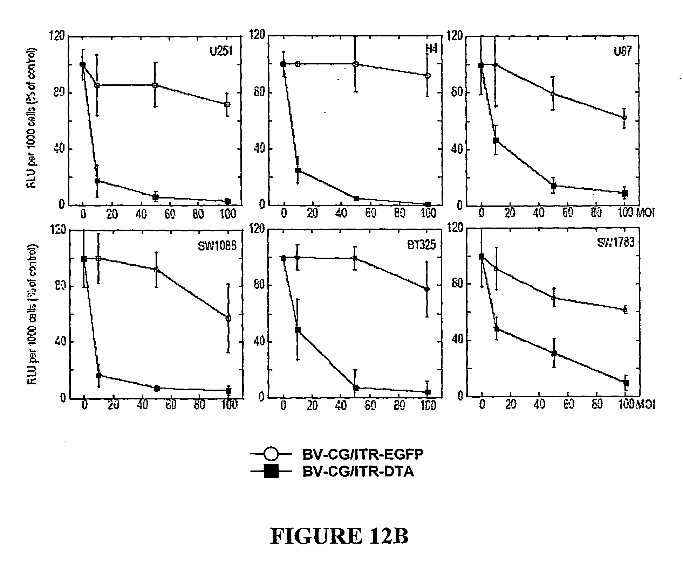

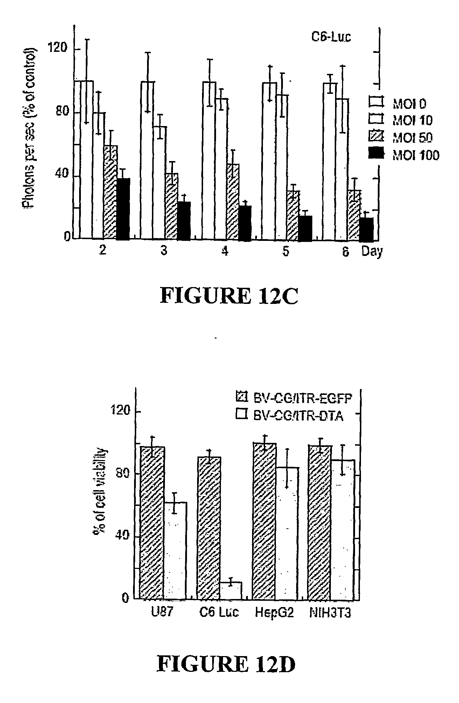

**Figure 12**. *In vitro* effects of baculovirus vectors carrying the DT-A gene. Cells were transduced in quadruplicate. A: RT-PCR analysis of DT-A expression in U251 cells 48 h after transduction at MOI of 100. B: Protein synthesis inhibition, as demonstrated by decrease of luciferase activity, was assayed 48 h after transduction with increased MOI from 10 to 100 in 6 glioma cell lines. C: After transduction of BV-CG/ITR-DTA in C6-Luc cells, time-dependent effects over 6 days were examined using the IVIS imaging system. D: Cell viability was determined by MTT assay 6 days after viral infection at an MOI of 100 in 4 cell lines.

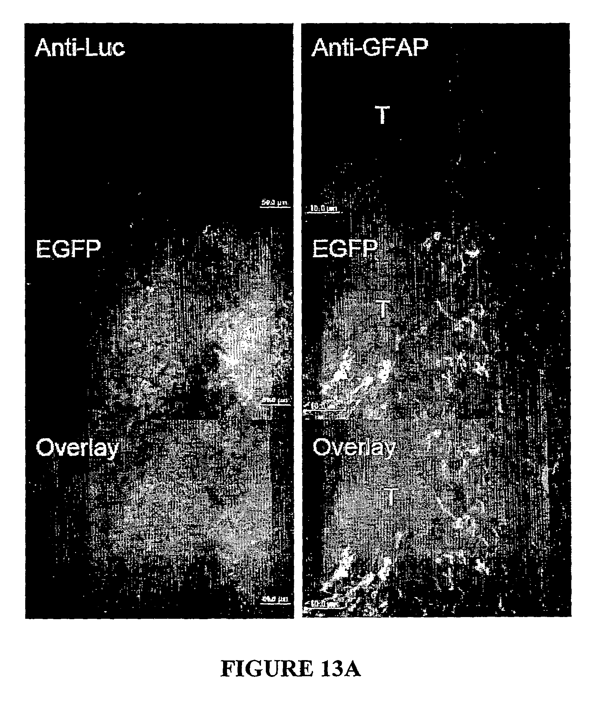

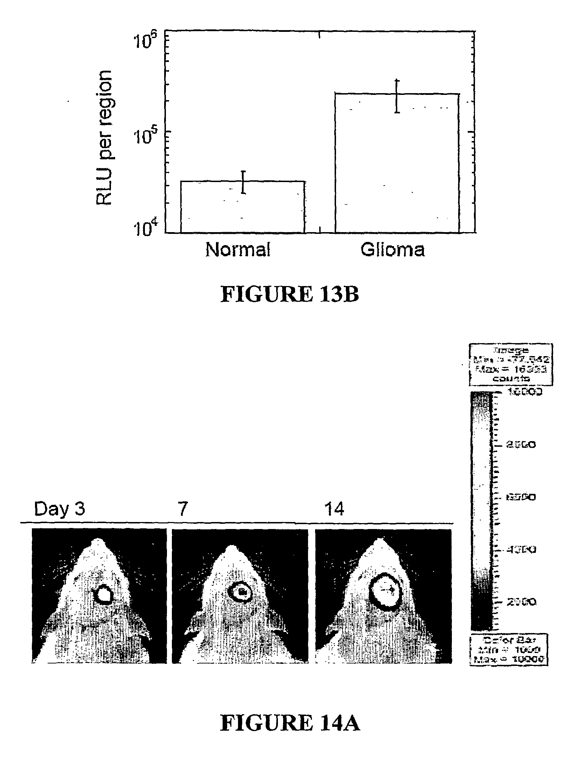

**Figure 13**. *In vivo* transgene expression in gliomas mediated by baculovirus carrying the hybrid CMV E/GFAP promoter and ITRs. A: BV-CG/ITR-EGFP was injected into the rat striatum that was inoculated with C6-luc glioma cells 3 days earlier. Immunostaining was carried out to show C6 cells and nearby astrocytes, while EGFP expression could be visually detected under a fluorescent microscope without immunostaining. The left panel, immunostaining with antibodies against luciferase to show glioma tissues. The right panel, immunostaining with antibodies against GFAP to show reactive gliosis near the inoculated tumor cells (T). B: Quantification of transgene expression. BV-CG/ITR-Luc was injected into the rat striatum that was inoculated with C6 glioma cells (without the luciferase gene) 3 days ago, and the contralateral normal striatum. Luciferase expression was measured 2 days after the virus injection. The results are expressed in relative light units (RLU) per brain and presented as means with SD (n=4).

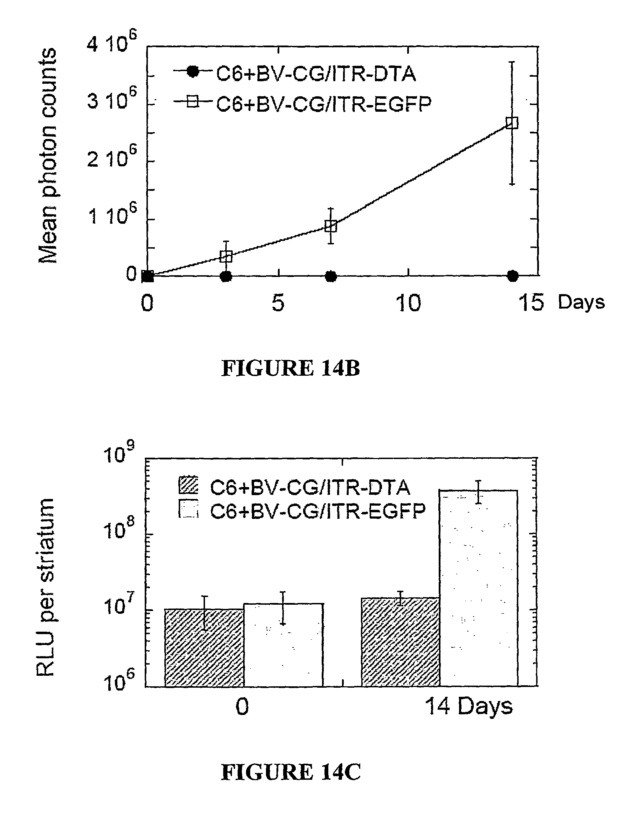

**Figure 14**. *In vivo* assays of C6 glioma growth in the rat brain. Anesthetized rats were inoculated with C6-luc cells to each side of the brain, followed by injection of BV-CG/ITR-DTA on the left side and BV-CG/ITR-EGFP on the right side 3 days later (designated as day 0). A: *In vivo* bioluminescent images of the brains with inoculated C6-luc cells 3, 7 and 14 days after virus injection in a living animal. The luminescent light emitted from the side injected with the control viruses was easily detected and increased over time. No light could be detected on the BV-CG/ITR-DTA injected side. B: Quantification of *in vivo* bioluminescence. Mean photon counts were quantified and are displayed over time. The data are presented as means with SD. Photon counts at day 0 were not much different form background bioluminescence. N = 6 at day 3 and 7 and n = 5 at day 14, as one rat died at day 12. C: Measurement of tumor growth by luciferase activity assays of brain tissues collected at day 0 (n = 3) and day 14 (n = 5). The results are expressed in relative light units (RLU) per brain and presented as means with SD.

**RESULTS**

**Effective transduction of glioma cells by baculovirus vectors**: The transduction efficiency of baculoviruses varies in different mammalian cells (Kost TA and Condreay JP. Trends Biotechnol (2002) 20:173-80). Therefore, we first tested baculovirus-mediated gene transfer in seven glioma cell lines, namely C6, H4, SW1088, SW1783, U87, U251 and BT325, with two baculovirus vectors, BV-CMV-Luc and BV-CMV-EGFP, containing a luciferase and an EGFP gene, respectively, under the control of the ubiquitous CMV promoter. The broad activity of the CMV promoter facilitates the comparison of gene expression levels between glioma cell lines with different levels of malignancy. As demonstrated with quantitative luciferase activity assays, BV-CMV-Luc dose-dependently transduced all the tested glioma cell lines with similar efficiency, except a somewhat higher level of transduction in U87 and a lower level in H4 at a multiplicity of infection (MOI) of 100 plaque-forming units (pfu) of viral particles per cell (Fig. 10A). Using BV-CMV-EGFP, we visually detected transgene expression in glioma cells as early as 4 to 6 hr after viral transduction. Flow cytometry analysis of these cells 24 hr after transduction indicated the percent transduced cells with intense green color ranging from 30 to 70% at a viral MOI of 100 (Fig. 10B). Increasing the MOI value to 200 resulted in only 10% improvement, indicating that the transduction efficiency in glioma cells had reached a plateau at an MOI around 100.

**Improved gene transfer into glioma cells by baculovirus vectors with modified GFAP promoters**: Using cell-type specific promoter to drive the expression of a therapeutic gene would ensure therapeutic efficacy in the cells of interest while limiting side effects in non-target cells. We constructed a baculovirus vector, BV-GFAP-Luc, with a GFAP promoter to drive the expression of a luciferase in order to restrict gene expression in glial cells in the brain (Fig. 11A). This vector, however, provided only low levels of transgene expression in the tested glioma cells, being 10 to several hundred-fold lower than those from the baculovirus vector with the CMV promoter (BV-CMV-Luc, Fig. 11A). We then placed two modified expression cassettes into the baculoviral vector (Fig. 11A). In one of the modified vectors the CMV enhancer from human cytomegalovirus was inserted upstream to the GFAP promoter (BV-CMV E/GFAP-Luc) and in another the ITR sequences from AAV were used to flank the luciferase gene and the hybrid CMV E/GFAP promoter (BV-CG/ITR-Luc). As shown in Figure 11A, the CMV enhancer in the context of baculovirus significantly improved the strength of the GFAP promoter in all tested glioma cell lines, resulting in the levels of gene expression close to those driven by the CMV promoter. With AAV ITR flanking, further improvement in gene expression, by at least 10-fold relative to BV-CMV E/GFAP-Luc, was observed. In 5 out of 7 tested glioma cell lines, the levels from BV-CG/ITR-Luc were even higher than those provided by BV-CMV-Luc (Fig. 11A).

In two non-glioma cell lines, HepG2 and NIH3T3, BV-CMV-luc provided transgene expression levels similar to those in the glioma cell lines infected with the same viral vector (Fig. 11A). In contrast, BV-GFAP-Luc, BV-CMV E/GFAP-Luc and BV-CG/ITR-Luc displayed significantly lower activities than BV-CMV-Luc in HepG2 and NIH3T3 cells. As BV-CMV-Luc transduced the two non-glioma cell lines and those glioma cell lines with similar efficiency, the low levels of gene expression from the three viral vectors with the GFAP promoter in HepG2 and NIH3T3 cells was less likely caused by the difference in cellular uptake of viruses. These findings indicate the cell-type specificity of the modified gene expression cassettes with the GFAP promoter.

While the enzymatic activity of luciferase is a sensitive quantitative parameter to evaluate gene transfer efficiency, especially in a study to compare the strength of the promoter in a particular cell line, the number of EGFP positive cells would be more indicative of the percentage of transduced cells. Therefore, we constructed a baculoviral vector with the hybrid CMV E/GFAP promoter flanked by AAV ITR sequences to drive the expression of the EGFP gene (BV-CG/ITR-EGFP). Flow cytometry analysis demonstrated a significant improvement in transduction efficiency over the experiments using the CMV promoter in all tested cell lines, with the percentage of transduced glioma cells ranging from 54% in SW1088 to 98% in C6 cells (Fig. 11B).

**A DT-A expressing baculovirus construct inhibits protein synthesis and cell growth in cultured glioma cells**: To explore the feasibility of using baculoviruses for glioma therapy, a new recombinant baculovirus vector was constructed containing a cDNA encoding DT-A under the control of the hybrid CMV E/GFAP promoter and flanked by the AAV ITRs (BV-CG/ITR-DTA). Although DT-A is highly toxic, viral particles with high titers were successfully generated in Sf9 cells, most likely due to the tight transcriptional control of the GFAP promoter in the insect cells. The expression of the DT-A gene from the baculovirus vector was confirmed by RT-PCR with DT-A gene-specific primers in U251 glioma cells (Fig. 12A).

We then tested whether BV-CG/ITR-DTA can block protein synthesis in glioma cells by evaluating its effect on expression of luciferase transgene. Six glioma cell lines were transduced with BV-CMV-Luc together with either BV-CG/ITR-DTA or BV-CG/ITR-EGFP. Forty-eight hours later, cell lysates were prepared and assayed for luciferase activity. Even at a viral MOI of 10, significant decrease in luciferase activity was observed in all the tested glioma cell lines, with about 50% inhibition in BT325 to almost 90% inhibition in SW1088 cells (Fig. 12B). Transduction of glioma cells with a higher concentration of the control virus decreased luciferase activity in some cell lines but the more dramatic effects were obvious when the viruses expressing DT-A were used.

To examine the DT-A inhibition effect over time, we used the sensitive IVIS cooled CCD camera system to monitor progressive change in luciferase activity over 6 days in stable rat C6 glioma cells engineered to express firefly luciferase (C6-Luc). After transduction of C6-Luc cells with BV-CG/ITR-DTA, luciferase activity decreased from 59% of the control on day 2 to 32% on day 6 at a viral MOI of 50 and from 38% of the control on day 2 to 14% by day 6 at an MOI of 100 (Fig. 12C). Inhibition of protein synthesis might eventually result in cell death, which would be another cause of deduction in luciferase activity on day 6. Transduction with an MOI of 10 resulted in 20 to 30 % of inhibition on day 2 and 3 but not at later time points, probably due to rapid cell growth of non-transduced C6-Luc cells (Fig. 12C).

To test the direct cytotoxic effect of BV-CG/ITR-DTA, cell viability assays were performed in C6-Luc and human U87 glioma cells, as well as two non-glioma HepG2 and NIH3T3 cell lines. These cells were transduced with BV-CG/ITR-DTA or BV-CG/ITR-EGFP at an MOI of 100 pfu of viral particles per cell and the cell viability assay was carried out 6 days after viral transduction. As shown in Fig. 12D, transduction of the viruses with the DT-A expression cassette resulted in 90% of growth inhibition in C6-luc cells and 40% in U87 glioma cells but not obviously in HepG2 and NIH3T3 cells.

**Baculovirus-mediated expression of reporter genes in glioma cells inoculated in the rat brain**: To investigate the possibility of using baculoviruses for *in vivo* glioma gene therapy, we tested in an animal glioma xenograft model the expression of the EGFP gene and the luciferase gene. In the first experiment, rat C6-Luc glioma cells were inoculated into the rat striatum and allowed to grow for 3 days to establish a glioma xenograft model. BV-CG/ITR-EGFP was then injected into the glioma tumor at the same position and immunostaining was carried out 2 days later. As shown in Fig. 13A, luciferase-positive C6 cells expressed detectable EGFP. In the sections immunostained with antibodies against GFAP, the solid gliomas were delineated by a rim of reactive astrocytes with strong GFAP singnals. Many of these reactive astrocytes also expressed high levels of EGFP, displaying bright green fluorescence (Fig. 13A, the right panel). We did not observe any EGFP positive cells in the normal region outside the gliosis rim.

In the second *in vivo* experiment, we aimed to compare the transgene expression levels in glioma cells and normal astrocytes. We inoculated C6 glioma cells without the luciferase gene into the one side of the rat striatum. Three days later, we injected the same amount of BV-CG/ITR-Luc into the C6 cells-inoculated brain region and the contralateral side of the rat brain respectively. Two days after viral injection we collected the brain tissues and measured luciferase activity. We detected a 10-fold higher level of luciferase expression in C6-inoculated brain region than that in normal brain (Fig. 13B).

**Inhibition of glioma xenograft growth in the rat brain by the DT-A expressing baculovirus construct**: Having shown the potent cytotoxicity in cultured glioma cells induced by baculoviruses with the DT-A gene and the efficient transduction of baculovirus in glioma xenograft, we then explored the *in vivo* antiglioma effect of BV-CG/ITR-DTA in the rat brain. Rat C6-Luc glioma cells were used to facilitate non-invasive quantitative evaluation of tumor growth in living animals with the IVIS *In Vivo* Imaging System. C6-luc cells were inoculated into the striatum on both sides. Three days later, BV-CG/ITR-DTA was injected into the left side and BV-CG/ITR-EGFP as the control into the right side. Tumor cell growth in the rat brain was monitored 0, 3, 7 and 14 days after virus injection. Fig. 14A shows the easily detected luminescent activity from the inoculated C6-luc cells on the control side from day 3 onward and the continuous increase of the luminescent intensity during a 14-day experiment period in one rat. On the DT-A injected side of the same animal, there was no detectable luciferase activity. Quantitative results from these rats were summarized in Fig. 14B, showing an obvious growth inhibition of C6 glioma cells by just one injection of BV-CG/ITR-DTA.

Tumor growth in the brain was also examined by measuring tissue luciferase activity. Brain tissues from both sides of C6-luc inoculated rats were collected at day 0 (3 day after tumor cell inoculation) and day 14 after intratumor injection of baculoviruses. At the time of viral injection (day 0), luciferase activities from two sides of the brain were very close. Two weeks after viral injection, the activity on the control side injected with BV-CG/ITR-EGFP was 30-fold higher than that on the BV-CG/ITR-DTA-injected side (Fig. 14C), indicating that the DT-A expression from the baculoviral vector effectively inhibited growth of tumor cells in the brain.

**EXAMPLE 3**

**MATERIAL AND METHODS**

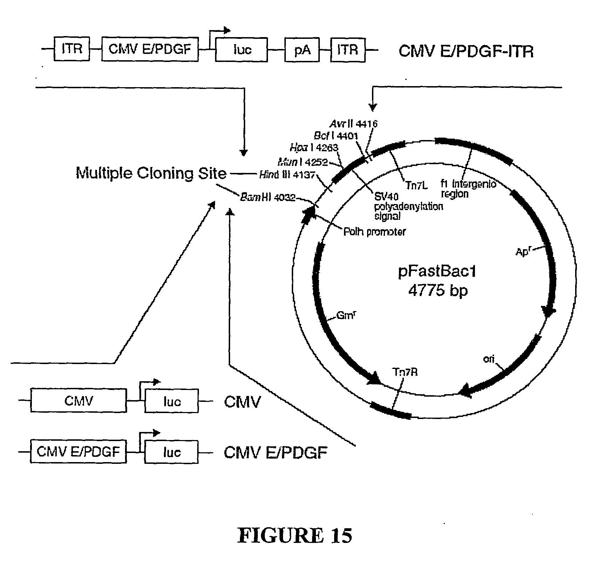

**Construction of recombinant baculoviral vectors**: pFastBac1 plasmid (Invitrogen, Carlsbad, CA) was used as the transfer plasmid for the construction of recombinant baculoviral vectors (Fig. 15). The CMV promoter or the hybrid CMV enhancer (CMV E)/PDGF promoter (Liu et al., (2004) Gene Ther. 11, 52-60) was inserted into pFastBac1 between the *Not*I and *Xba*I sites. A luciferase gene, used as the reporter gene for all the constructions tested in this study, was inserted downstream of the promoters, between the *Xho*I and *Hind*III sites. To construct a baculoviral vector with the ITRs of AAV, an expression cassette containing a multiple cloning site, a reporter gene encoding luciferase, a simian virus 40 (SV40) poly(A) signal, and two ITR sequences at both ends was amplified from pAAV plasmid (Wang et al., (2005) J. Gene Med. 7, 945-955) and inserted into pFastBac1 between the *Avr*II and *Sal*I sites. The CMV E/PDGF promoter was then inserted into the *Kpn*I and *Hind*III sites to drive the luciferase reporter gene. Recombinant baculoviral vectors carrying the above-described promoters, named BV-CMV, BV-CMV E/PDGF, and BVCMV E/PDGF-ITR, respectively, were produced and propagated in Sf9 insect cells according to the Bac-to-Bac baculovirus expression system manual (Invitrogen).

**Viral transduction and gene expression analysis**: For *in vitro* transduction, human NT-2 cells, mouse C17.2 cells, or rat PC12 cells were seeded in 48-well plates at a density of 2 x 10⁴ cells per well. One hundred microliters of Dulbecco's modified Eagle's medium (DMEM) containing baculoviral vector was added to each well. One hour later, normal culture medium was added to the cells. Cells were further cultured for various periods of time before being collected for luciferase activity measurements.

For luciferase activity assays, cells were washed and permeabilized with 100 µl of reporter cell lysis buffer (Promega, Madison, WI). Luciferase activity in cell extracts was measured with a luciferase assay kit (Promega) in a single-tube luminometer (Lumat LB 9507; Berthold Technologies, Bad Wildbad, Germany) for 10 sec. The total protein concentration of each lysate was determined by DC protein assay (Bio-Rad, Hercules, CA). Results were expressed as relative light units (RLU) per milligram of total protein. Luciferase expression from *in vitro* experiments was analyzed statistically by Student *t* test.

For *in vivo* transduction, adult male Wistar rats (weighing 250-320 g) were used, four rats per time point. Rats were anesthetized by intraperitoneal injection of sodium pentobarbital (60 mg/kg body weight) and positioned in a stereotaxic instrument with the nose bar set at 0. Five microliters of baculoviral vector (5 x 10⁶ PFU) was injected at a speed of 0.5 µl/min into the striatum (anteroposterior [AP], +1.0 mm; mediolateral [ML], +2.5 mm; and dorsoventral [DV], -5.0 mm from bregma and dura), using a 10 µl Hamilton syringe connected with a 30-gauge needle. The needle was allowed to remain in place for another 5 min before being slowly retracted at the end of each injection.

For luciferase activity assays, rats were killed after an appropriate period of time. Tissue samples from different brain regions were collected and homogenized in phosphate-buffered saline (PBS; 100 µl of PBS per 50 mg of tissue) by sonication for 10 sec on ice. Homogenized tissues were then centrifuged at 13,000 rpm for 10 min at 4°C. For each sample, 10 µl of the supernatant was used for the luciferase activity assay as stated above. The relative light unit value from each tissue sample was calculated and the results were expressed as relative light units per region. Luciferase expression from *in vivo* experiments was analyzed statistically by Student t test. Immunohistochemical analysis of transduced brain tissues was carried out as reported previously (Li *et al.,* (2004), *supra*).

**Southern blotting**: For Southern blot analysis, NT-2 cells were infected with BV-CMV E/PDGF-ITR at a multiplicity of infection (MOI) of 100. Two and 7 days after infection, genomic DNA was extracted with a DNeasy tissue kit (Qiagen, Hilden, Germany) from the infected cells as well as from normal NT-2 cells that had not been virally infected (negative control). The genomic DNA samples were digested with *Xho*I before agarose gel electrophoresis, because the enzyme can release an 8.6-kb DNA fragment containing the whole ITR-flanked expression cassette from the genomic DNA of BV-CMV E/PDGF-ITR. After electrophoresis in 1% agarose gel, the digested DNA samples were transferred onto a nylon membrane, and detected by a digoxigenin (DIG)-labeled probe prepared from polymerase chain reaction (PCR) amplification of the luciferase reporter gene (1076-1318 nt from the initiating ATG).

**Quantitative real-time PCR**: NT-2 cells were infected with BV-CMV E/PDGF or BVCMV E/PDGF-ITR at an MOI of 25. Two days after infection, total RNA was extracted from infected NT-2 cells with an RNeasy mini kit (Qiagen). Identical RNA concentrations were used for reverse transcription. PCR primers were designed specifically to amplify a sequence of firefly luciferase gene and were obtained from Applied Biosystems (Foster City, CA). The forward primer used was 5'-CGCCCTGGTTCCTGGAA-3' **[SEQ ID NO: 7]** and the reverse primer was 5'-GGACATTTCGAAGTACTCAGCGTAA- 3' **[SEQ ID NO: 8]**. A custom TaqMan probe (5'-ATGTCCACCTCGATATGTG- 3') **[SEQ ID NO: 9]** containing a sequence complementary to the PCR product was used for detection. Quantitative real-time PCR was performed with 1 µl of the prepared cDNA. Default real-time PCR assay conditions were employed. All PCR amplifications were performed in triplicate. Actin was used as the housekeeping gene. Relative quantification of transcripts was done by the 2^{-ΔΔC} T method (Livak and Schmittgen, (2001) Methods 25, 402-408).

**DETAILED FIGURE LEGENDS**

**Figure 15**. Schematic diagram of the expression cassettes used in neuronal specific baculoviral vectors. PFastBac1 plasmids were used for the construction of baculovirus shuttle vectors in *E. coli* host strain DH10Bac. The produced recombinant baculovirus shuttle vectors were then transfected into Sf9 cells to produce recombinant baculoviruses. CMV, human cytomegalovirus immediately-early gene promoter; CMV E, enhancer of the human cytomegalovirus immediately-early gene; PDGF, platelet-derived growth factor β-chain promoter; ITR, AAV inverted terminal repeat; *luc,* luciferase gene; pA, SV40 poly(A) signal.

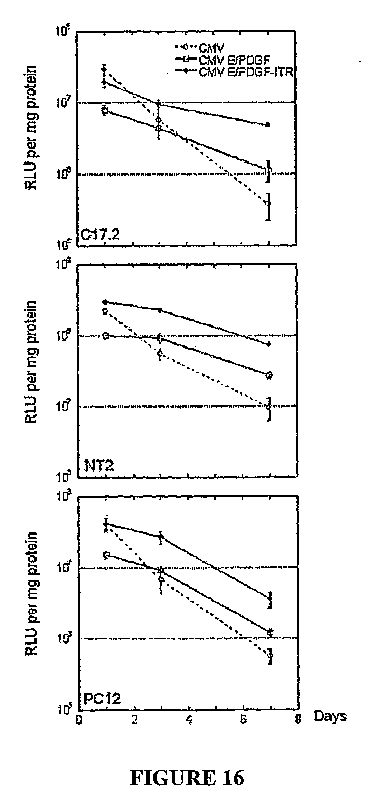

**Figure 16****.** Kinetics of luciferase expression in cultured cells infected with recombinant baculoviruses. The results are presented as relative light units (RLU) per milligram of total protein (means ± SD, *n* = 4).

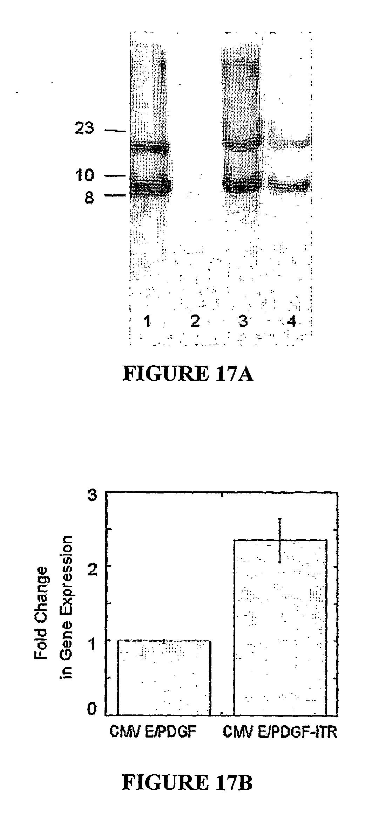

**Figure 17****.** Southern blot **(A)** and quantitative real-time PCR **(B)** analyses of baculovirus-infected NT-2 cells. **(A)** Cells were infected with BV-CMV E/PDGF-ITR. Lane 1, DNA of BV-CMV E/PDGF-ITR; lane 2, DNA extracted from normal NT-2 cells without virus infection; lanes 3 and 4, DNA extracted from NT- 2 cells 2 and 7 days, respectively, after BV-CMV E/PDGF-ITR infection. The positions of molecular size standards (kb) are indicated. **(B)** Real-time PCR results of luciferase gene expression from CMV E/PDGF and CMV E/PDGF-ITR. Values represent the fold change in luciferase gene expression, normalized to actin gene expression and relative to luciferase expression from CMV E/PDGF (means ± SD, *n* = 3).

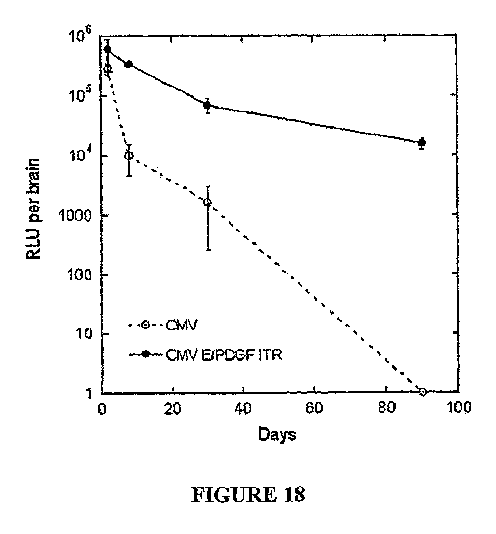

**Figure 18****.** Kinetics of luciferase expression in rat brain after a single injection of recombinant baculovirus. Values are expressed as relative light units per brain and are presented as means ± SD (*n* = 4).

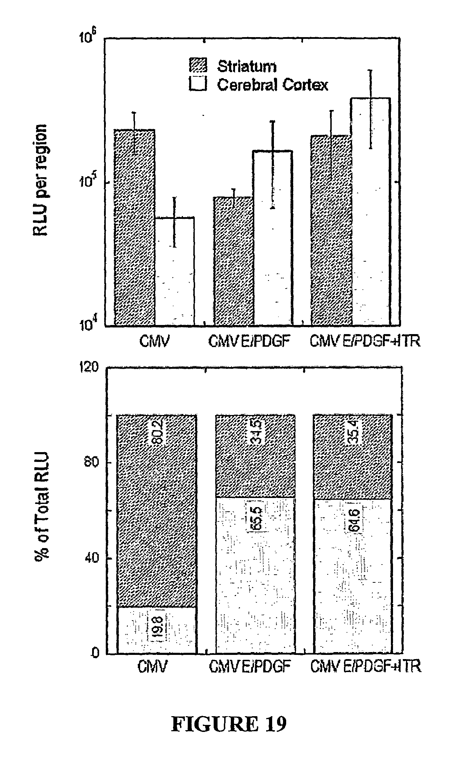

**Figure 19****.** PDGF promoter activity in neurons as measured by luciferase expression in a brain region remote from the injection site. Two days after viral vector injection, rat brain tissue samples from striatum (injection site) and cerebral cortex tissues (remote region) were collected. Their luciferase activities were measured and expressed as relative light units per region (means ± SD, *n* = 4).

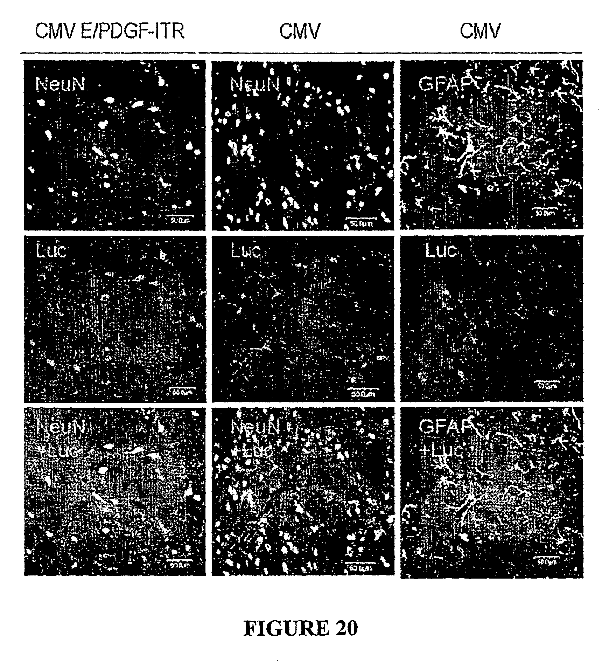

**Figure 20****.** Immunohistological analysis of rat brains injected with recombinant baculoviruses. Five microliters (1 x 10⁸ PFU) of BV-CMV E/PDGF-ITR or BV-CMV was injected into the striatum of adult male Wistar rats. Two days after injection, brain samples were collected and frozen coronal sections of each brain, within 0.5 mm of the needle track, were cut at a thickness of 30 µm for.free-floating immuno-staining with antibodies against neuronal nuclear protein (NeuN) or glial fibrillary acidic protein (GFAP).

**RESULTS**

Transgene : expression mediated by baculoviral vectors was first examined *in vitro* in three neural cell lines, namely, mouse C17.2, rat PC12, and human NT-2 cells. Cells were infected with the three vectors at an MOI of 25. Luciferase expression in the transduced cells was measured 1, 3, and 7 days after infection (Fig. 16). On day 1, BV-CMV and BV-CMV E/PDGF-ITR produced 3- to 5-fold higher levels of gene expression than did BV-CMV E/PDGF in the three cell lines tested. BV-CMVmediated gene expression then decreased continuously over almost two orders of magnitude within 7 days, by day 7 becoming about 3-fold lower than that mediated by. BV-CMV E/PDGF (*p* < 0.05). BV-CMV E/PDGF-ITR still provided a level of gene expression severalfold higher than BV-CMV E/PDGF at the two later time points and performed significantly better than BV-CMV, mediating 6-, 8-, and 13-fold higher levels of expression in PC12, NT-2, and C17.2 cells, respectively, on day 7 (*p* < 0.001). This time course study was repeated 2 months after the initial study, using a new preparation of the three viral vectors, and similar improvements were observed (data not shown). In an effort to understand the mechanism underlying the extended gene expression provided by BV-CMV E/PDGF-ITR, we performed Southern blot analysis of genomic DNA extracted from NT-2 cells infected with BV-CMV E/PDGF-ITR. A band with a size close to 8 kb, as expected after *Xho*I digestion of baculovirus genomic DNA, was clearly detectable in the positive control of baculovirus genomic DNA and in DNA extracted from baculovirus-infected NT-2 cells, but not in DNA extracted from the negative control of normal NT-2 cells without viral infection (Fig. 17A). Probably because of the degradation of viral DNA within the cells, the intensity of the band decreased from day 2 to day 7 in infected NT-2 cells (Fig. 17A). Another DNA band with a larger size was also detected in the baculovirus genomic DNA and in DNA extracted from the baculovirus- infected NT-2 cells, possibly resulting from the incomplete digestion of baculovirus genomic DNA. These results suggest that the BV-CMV E/PDGF-ITR genome remained episomal after being taken in by NT-2 cells and that neither chromosomal integration nor the formation of high molecular weight concatamers of ITR-flanking expression cassette occurred within the experimental period. We further performed quantitative real-time PCR to compare the levels of luciferase mRNA provided by BV-CMV E/PDGF and BV-CMV E/PDGF-ITR. We observed that the mRNA level from CMV E/PDGF-ITR was 2.36-fold higher than that from CMV E/PDGF (Fig. 17B), indicating that transcription enhancement could be one possible reason for the improved gene expression observed in the results shown in Fig. 16.

*In vivo* gene transfer to rat brain was examined after stereotaxic injection of 5 µl (5 x 10⁶ PFU) of BV-CMV or BV-CMV E/PDGF-ITR into rat striatum, followed by analysis of luciferase expression at various time points up to 90 days (Fig. 18). BV-CMV mediated an initially high level of luciferase expression, which dropped sharply and was undetectable on day 90. Luciferase expression achieved with BV-CMV E/PDGF-ITR also decreased over time but at a much slower rate, being significantly higher than that of BV-CMV from day 8 onward. Especially on day 90, the last time point in the study, a relatively high level of gene expression from BV-CMV E/PDGF-ITR was still detectable.

Our previous study demonstrated that baculoviruses, after being internalized by nerve terminals at an injection site, could migrate by axonal transport to neuronal cell bodies located in a remote region (Li *et al.,* (2004), *supra*). This feature offers a unique opportunity to study the specificity and activity of a promoter in neurons, as gene expression detected in a region remote from the injection site would be a result of the axonal transport of baculoviral vectors and represent purely neuronal expression. In the current study, we analyzed gene expression mediated by the three baculoviral vectors in the striatum, the injection site, and in the cerebral cortex, a remote region where some neurons have long axons projecting to the striatum. We observed that the luciferase activity from BV-CMV was detected mainly at the injection site (80% of total activity), whereas the majority of the luciferase activity, about 65% of total activity, provided by the two viral vectors containing the neuron-specific CMV E/PDGF promoter was detected in the remote region (Fig. 19), suggesting that the hybrid PDGF promoter displayed a higher activity in neurons than did the CMV full-length promoter. When comparing the two viral vectors with the hybrid PDGF promoter, we observed that although ITR flanking increased gene expression levels in both the striatum and cerebral cortex, it did not alter the ratio of gene expression in these two regions. This finding suggests that ITR flanking does not affect the specificity of the PDGF promoter.

Neuronal specificity of the baculoviral vectors was further examined at the injection site by immunohistological staining. Two days after injection of 5 µl (1 x 10⁸ PFU) of BV-CMV E/PDGF-ITR or BV-CMV into rat striatum, brain tissue sections were collected for double immunostaining. Anti-luciferase antibodies were used to visualize transduced cells, and antineuronal nuclear protein (NeuN) and anti-glial fibrillary acidic protein (GFAP) antibodies were used to show neurons and astroglial cells, respectively. Cell counting on the tissue sections around the injection site revealed that in BV-CMV E/PDGFITR-injected samples, nearly 80% of transduced cells in the striatum were positively stained with NeuN antibodies (512 double-stained cells versus 648 luciferase-expressing cells). Among BV-CMV-transduced cells, only 5% were identified as neurons (38 double-stained cells versus 820 luciferase-expressing cells) and most of the transduced cells displayed morphology of astroglial cells and were positively stained with antibodies against GFAP (Fig. 20).

As can be understood by a skilled person, many modifications to the exemplary embodiments described herein are possible. The invention, rather, is intended to encompass all such modifications within its scope, as defined by the claims.

Although various embodiments of the invention are disclosed herein, many adaptations and modifications may be made within the scope of the invention in accordance with the common general knowledge of those skilled in this art. Such modifications include the substitution of known equivalents for any aspect of the invention in order to achieve the same result in substantially the same way as defined by the claims. All technical and scientific terms used herein have the same meaning as commonly understood by one of ordinary skill in the art of this invention, unless defined otherwise.

## Claims

1. A baculovirus vector comprising an expression cassette, the expression cassette comprising:
a glial fibrillary acidic protein (GFAP) promoter operably linked to a heterologous enhancer;
one or both of a coding sequence operably linked to the GFAP promoter and a cloning site for inserting a coding sequence operably linked to the GFAP promoter; and
a pair of viral inverted terminal repeats from adeno-associated virus, said pair of viral inverted terminal repeats flanking at least the operably linked GFAP promoter and said one or both of the coding sequence and the cloning site;
wherein the heterologous enhancer increases the glial-specific transcriptional activity of the GFAP promoter.

2. The baculovirus of claim 1 wherein the pair of viral inverted terminal repeats also flanks the heterologous enhancer.

3. The baculovirus vector of claim 1 or claim 2 wherein the GFAP promoter comprises the sequence set forth in SEQ ID NO: 1 or SEQ ID NO: 2.

4. The baculovirus vector of any one of claims 1 to 3 wherein the heterologous enhancer is a viral enhancer.

5. The baculovirus vector of claim 4 wherein the viral enhancer is cytomegalovirus immediate early enhancer or SV40 enhancer.

6. The baculovirus vector of claim 5 wherein the cytomegalovirus immediate early enhancer comprises the region -568 to -187 base pairs relative to the TATA box of the cytomegalovirus immediate early promoter.

7. The baculovirus vector of claim 6 wherein the cytomegalovirus immediate early enhancer has the sequence set forth in SEQ ID NO: 3.

8. The baculovirus vector of any one of claims 1 to 7 wherein the heterologous enhancer is operably linked upstream to the GFAP promoter.

9. The baculovirus vector of any one of claims 1 to 8 wherein the inverted terminal repeats comprise the sequence set forth in SEQ ID NO: 4.

10. The baculovirus vector of any one of claims 1 to 9, comprising the sequence as set forth in SEQ will NO: 5 upstream of the coding sequence or cloning site and the sequence as set forth in SEQ ID NO: 6 downstream of the coding sequence or cloning site.

11. The baculovirus vector of any one of claims 1 to 10 wherein the coding sequence encodes a therapeutic product.

12. The baculovirus vector of claim 11 wherein the therapeutic product is a protein, a peptide, a ribozyme, a small interfering RNA, a microRNA or an antisense RNA.

13. The baculovirus vector of claim 12 wherein the therapeutic product is a protein or peptide involved in the treatment of a neural-related disorder.

14. The baculovirus vector of claim 13 wherein the neural-related disorder is a neuronal cancer, a medulloblastoma, a neuroblastoma, a glioma, an astrocytoma, a neurodegenerative disorder, Alzheimer's disease, Parkinson's disease, epilepsy or damage as a result of alcohol exposure.

15. The baculovirus vector of claim 13 or 14 wherein the therapeutic product is a neurotrophic factor, a growth factor, an anti-apoptotic protein, a cytotoxin, an apoptotic protein, a tumour suppressor protein, an immunomodulator protein, an oncoprotein, an antibody or an anti-angiogenic protein.

16. The baculovirus vector of claim 15 wherein the therapeutic product is p53 or a p53 pathway protein.

17. The baculovirus vector of claim 15 wherein the therapeutic product is DT-A bacterial protein.

18. An in vitro method of expressing a nucleic acid in a glial cell or in a cell derived from a glial cell, comprising transfecting in vitro a glial cell or a cell derived from a glial cell with the baculovirus vector as defined in any one of claims 1 to 17.

19. A baculovirus vector as defined in any one of claims 1 to 17 for use in a method of treating a neural-related disorder in a subject, the method comprising expressing a nucleic acid in a glial cell or in a cell derived from a glial cell, wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

20. A baculovirus vector for use in a method of treating a neural-related disorder in a subject, the baculovirus vector comprising an expression cassette, the expression cassette comprising:
a glial fibrillary acidic protein (GFAP) promoter operably linked to a heterologous enhancer;
a coding sequence operably linked to the GFAP promoter; and
a pair of viral inverted terminal repeats from adeno-associated virus,
said pair of viral inverted terminal repeats flanking at least the operably linked GFAP promoter and coding sequence;
wherein the heterologous enhancer increases the glial-specific transcriptional activity of the GFAP promoter;
and wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

21. Use of a baculovirus vector for preparation of a medicament for treating a neural-related disorder in a subject, the baculovirus vector comprising an expression cassette, the expression cassette comprising:
a glial fibrillary acidic protein (GFAP) promoter operably linked to a heterologous enhancer;
a coding sequence operably linked to the GFAP-specific promoter; and
a pair of viral inverted terminal repeats from adeno-associated virus,
said pair of viral inverted terminal repeats flanking at least the operably linked GFAP promoter and coding sequence;
wherein the heterologous enhancer increases the glial-specific transcriptional activity of the GFAP promoter;
and wherein the neural-related disorder is a neuronal cancer, a medulloblastoma, a neuroblastoma, a glioma, an astrocytoma, a neurodegenerative disorder, Alzheimer's disease, Huntington's disease, Parkinson's disease, epilepsy, stroke, ischemia, head or spinal cord trauma, amyotrophic lateral sclerosis, a neurogenetic disorder or damage as a result of alcohol exposure.

22. The baculovirus vector of claim 20 or the use of claim 21 wherein the expression cassette is the expression cassette as defined in any one of claims 1 to 17.

23. The baculovirus vector of claim 20 or 22, or the use of claim 21 or 22, wherein the neural-related disorder is a neuronal cancer, a medulloblastoma, a neuroblastoma, a glioma, an astrocytoma, a neurodegenerative disorder, Alzheimer's disease, Parkinson's disease, epilepsy or damage as a result of alcohol exposure.

24. A transgenic cell comprising the baculovirus vector as defined in any one of claims 1 to 17.

25. A non-human animal comprising the baculovirus vector as defined in any one of claims 1 to 17.

26. A pharmaceutical composition comprising the baculovirus vector as defined in any one of claims 1 to 17.

## Patentansprüche

1. Baculovirusvektor, umfassend eine Expressionskassette, wobei die Expressionskassette umfasst:
einen sauren Gliafaserprotein(GFAP)-Promoter, der in operativer Weise mit einem heterologen Verstärker verbunden ist;
ein oder beide einer Kodiersequenz, die in operativer Weise mit dem GFAP-Promoter verbunden ist/sind und eine Klonierungsseite zum Insertieren einer Kodiersequenz,
die in operativer Weise mit dem GFAP-Promoter verbunden ist;
und
ein Paar viral invertierter terminaler Wiederholungseinheiten von Adeno-assoziierten Virus, wobei das genannte Paar der viral invertierten terminalen Wiederholungseinheiten wenigstens den in operativer Weise verbundenen GFAP-Promoter und die genannte eine oder beide der Kodiersequenz/Kodiersequenzen und die Klonierungsseite flankiert;
wobei der heterologe Verstärker die Glia-spezifische transkriptionelle Aktivität des GFAP-Promoters erhöht.

2. Baculovirusvektor nach Anspruch 1, wobei das Paar an viral invertierten terminalen Wiederholungseinheiten auch den heterologen Verstärker flankiert.

3. Baculovirusvektor nach Anspruch 1 oder Anspruch 2, wobei der GFAP-Promoter die Sequenz umfasst, die in SEQ ID NO: 1 oder SEQ ID NO:2 angegeben ist.

4. Baculovirusvektor nach einem der Ansprüche 1 bis 3, wobei der heterologe Verstärker ein viraler Verstärker ist.

5. Baculovirusvektor nach Anspruch 4, wobei der virale Verstärker ein Cytomegalovirus-Immediate-Early-Verstärker oder ein SV40-Verstärker ist.

6. Baculovirusvektor nach Anspruch 5, wobei der Cytomegalovirus-Immediate-Early-Verstärker die Region -568 bis -187-Basenpaare relativ zu der TATA-Box des Cytomegalovirus-Immediate-Early-Verstärkers umfasst.

7. Baculovirusvektor nach Anspruch 6, wobei der Cytomegalovirus-Immediate-Early-Verstärker die Sequenz aufweist, die in SEQ ID NO:3 angegeben ist.

8. Baculovirusvektor nach einem der Ansprüche 1 bis 7, wobei der heterologe Verstärker in operativer Weise stromauf mit dem GFAP-Promoter verbunden ist.

9. Baculovirusvektor nach einem der Ansprüche 1 bis 8, wobei die invertierten terminalen Wiederholungseinheiten die Sequenz umfassen, die in SEQ ID NO: 4 angegeben ist.

10. Baculovirusvektor nach einem der Ansprüche 1 bis 9, umfassend die Sequenz, die in SEQ ID NO: 5 stromauf der Kodiersequenz oder der Klonierungsseite und die Sequenz, die in SEQ ID NO: 6 stromab der Kodiersequenz oder der Klonierungsseite angegeben ist.

11. Baculovirusvektor nach einem der Ansprüche 1 bis 10, wobei die Kodiersequenz ein therapeutisches Produkt kodiert.

12. Baculovirusvektor nach Anspruch 11, wobei das therapeutische Produkt ein Protein, ein Peptid, ein Ribozym, eine kurze interferierende RNA, eine Mikro-RNA oder eine anitsense-RNA ist.

13. Baculovirusvektor nach Anspruch 12, wobei das therapeutische Produkt ein Protein oder Peptid ist, das bei der Behandlung einer nervenbedingten Störung involviert ist.

14. Baculovirusvektor nach Anspruch 13, wobei die nervenbedingte Störung ein neuronaler Krebs, ein Medullablastom, ein Neuroblastom, ein Gliom, ein Astrocytom, eine neurodegenerative Störung, Alzheimer-Krankheit, Parkinson-Krankheit, Epilepsie oder eine Schädigung als eine Folge von Alkoholaufnahme ist.

15. Baculovirusvektor nach Anspruch 13 oder 14, wobei das therapeutische Produkt ein neuroptropher Faktor, ein Wachstumsfaktor, ein anti-apoptotisches Protein, ein Cytotoxin, ein apoptotisches Protein, ein Tumorsuppressorprotein, ein Immunmodulatorprotein, ein Onkoprotein, ein Antikörper oder ein anti-angiogenes Protein ist.

16. Baculovirusvektor nach Anspruch 15, wobei das therapeutische Produkt p53 oder ein p53-Pathwayprotein ist.

17. Baculovirusvektor nach Anspruch 15, wobei das therapeutische Produkt DT-A-bakterielles Protein ist.

18. In Vitro-Verfahren zum Exprimieren einer Nukleinsäure in eine Gliazelle oder in eine Zelle, abgeleitet von einer Gliazelle, umfassend Transfizieren in vitro eine Gliazelle oder eine Zelle, abgeleitet von einer Gliazelle mit dem Baculovirusvektor, wie in einem der Ansprüche 1 bis 17 definiert.

19. Baculovirusvektor nach einem der Ansprüche 1 bis 17 für die Verwendung in einem Verfahren zum Behandeln einer nervenbedingten Störung in einem Individuum, wobei das Verfahren umfasst das Exprimieren einer Nukleinsäure in eine Gliazelle oder in eine Zelle, abgeleitet von einer Gliazelle, wobei die nervenbedingte Störung ein neuronaler Krebs, ein Medullablastom, ein Neuroblastom, ein Gliom, ein Astrocytom, eine neurodegenerative Störung, Alzheimer-Krankheit, Huntington-Krankheit, Parkinson-Krankheit, Epilepsie, Schlaganfall, Ischämie, Kopf- oder Rückenmarkstrauma, amyotrophe Lateralsklerose, eine neurogenetische Störung oder eine Schädigung als eine Folge von Alkoholaufnahme ist.

20. Baculovirusvektor für die Verwendung in einem Verfahren der Behandlung einer nervenbedingten Störung in einem Individuum, wobei der Baculovirusvektor eine Expressionskassette umfasst, wobei die Expressionskassette umfasst:
einen sauren Gliafaserprotein(GFAP)-Promoter, der in operativer Weise mit einem heterologen Verstärker verbunden ist;
eine Kodiersequenz, die in operativer Weise mit dem GFAP-Promoter verbunden ist;
und
ein Paar viral invertierter terminaler Wiederholungseinheiten von Adeno-assoziierten Virus, wobei das genannte Paar der viral invertierten terminalen Wiederholungseinheiten wenigstens den in operativer Weise verbundenen GFAP-Promoter und die Kodiersequenz flankiert;
wobei der heterologe Verstärker die Glia-spezifische transkriptionelle Aktivität des GFAP-Promoters erhöht und
wobei die nervenbedingte Störung ein neuronaler Krebs, ein Medullablastom, ein Neuroblastom, ein Gliom, ein Astrocytom, eine neurodegenerative Störung, Alzheimer-Krankheit, Huntington-Krankheit, Parkinson-Krankheit, Epilepsie, Schlaganfall, Ischämie, Kopf- oder Rückenmarkstrauma, amyotrophe Lateralsklerose, eine neurogenetische Störung oder eine Schädigung als eine Folge von Alkoholaufnahme ist.

21. Verwendung eines Baculovirusvektor für die Herstellung eines Medikaments für die Behandlung einer nervenbedingten Störung in einem Individuum, wobei der Baculovirusvektor eine Expressionskassette umfasst, wobei die Expressionskassette umfasst:
einen sauren Gliafaserprotein(GFAP)-Promoter, der in operativer Weise mit einem heterologen Verstärker verbunden ist;
eine Kodiersequenz, die in operativer Weise mit dem GFAP-spezifischen Promoter verbunden ist;
und
ein Paar viral invertierter terminaler Wiederholungseinheiten von Adeno-assoziierten Virus, wobei das genannte Paar der viral invertierten terminalen Wiederholungseinheiten wenigstens den in operativer Weise verbundenen GFAP-Promoter und die Kodiersequenz flankiert;
wobei der heterologe Verstärker die Glia-spezifische transkriptionelle Aktivität des GFAP-Promoters erhöht und
wobei die nervenbedingte Störung ein neuronaler Krebs, ein Medullablastom, ein Neuroblastom, ein Gliom, ein Astrocytom, eine neurodegenerative Störung, Alzheimer-Krankheit, Huntington-Krankheit, Parkinson-Krankheit, Epilepsie, Schlaganfall, Ischämie, Kopf- oder Rückenmarkstrauma, amyotrophe Lateralsklerose, eine neurogenetische Störung oder eine Schädigung als eine Folge von Alkoholaufnahme ist.

22. Baculovirusvektor nach Anspruch 20 oder dessen Verwendung nach Anspruch 21, wobei die Expressionskassette die Expressionskassette ist, die in einem der Ansprüche 1 bis 17 definiert ist.

23. Baculovirusvektor nach Anspruch 20 oder 22 oder dessen Verwendung nach Anspruch 21 oder 22, wobei die nervenbedingte Störung ein neuronaler Krebs, ein Medullablastom, ein Neuroblastom, ein Gliom, ein Astrocytom, eine neurodegenerative Störung, Alzheimer-Krankheit, Parkinson-Krankheit, Epilepsie, oder eine Schädigung als eine Folge von Alkoholaufnahme ist.

24. Transgene Zelle, umfassend den Baculovirusvektor nach einem der Ansprüche 1 bis 17.

25. Nichtmenschliches Tier, umfassend den Baculovirusvektor nach einem der Ansprüche 1 bis 17.

26. Pharmazeutische Zusammensetzung, umfassend den Baculovirusvektor nach einem der Ansprüche 1 bis 17.

## Revendications

1. Vecteur baculovirus comprenant une cassette d'expression, la cassette d'expression comprenant :
un promoteur de la protéine acide fibrillaire gliale (GFAP) en liaison fonctionnelle avec un amplificateur hétérologue ;
l'un des deux, ou les deux, parmi une séquence codante en liaison fonctionnelle avec le promoteur de la GFAP et un site de clonage pour insérer une séquence codante en liaison fonctionnelle avec le promoteur de la GFAP ; et
une paire de répétitions terminales inversées virales d'un virus adéno-associé, ladite paire de répétitions terminales inversées virales flanquant au moins le promoteur de la GFAP en liaison fonctionnelle et ledit l'un des deux, ou les deux, parmi la séquence codante et le site de clonage ;
où l'amplificateur hétérologue augmente l'activité transcriptionnelle gliale-spécifique du promoteur de la GAFP.

2. Vecteur baculovirus selon la revendication 1, dans lequel la paire de répétitions terminales inversées virales flanque également l'amplificateur hétérologue.

3. Vecteur baculovirus selon la revendication 1 ou 2, dans lequel le promoteur de la GFAP comprend la séquence décrite dans SEQ ID NO: 1 ou SEQ ID NO: 2.

4. Vecteur baculovirus selon l'une quelconque des revendications 1 à 3, dans lequel l'amplificateur hétérologue est un amplificateur viral.

5. Vecteur baculovirus selon la revendication 4, dans lequel l'amplificateur viral est l'amplificateur précoce immédiat du cytomégalovirus ou l'amplificateur de SV40.

6. Vecteur baculovirus selon la revendication 5, dans lequel l'amplificateur précoce immédiat du cytomégalovirus comprend les paires de bases de la région -568 à -187 par rapport à la boîte TATA du promoteur précoce immédiat du cytomégalovirus.

7. Vecteur baculovirus selon la revendication 6, dans lequel l'amplificateur précoce immédiat du cytomégalovirus possède la séquence décrite dans SEQ ID NO: 3.

8. Vecteur baculovirus selon l'une quelconque des revendications 1 à 7, dans lequel l'amplificateur hétérologue est en liaison fonctionnelle en amont du promoteur de la GFAP.

9. Vecteur baculovirus selon l'une quelconque des revendications 1 à 8, dans lequel les répétitions terminales inversées comprennent la séquence décrite dans SEQ ID NO: 4.

10. Vecteur baculovirus selon l'une quelconque des revendications 1 à 9, comprenant la séquence telle que décrite dans SEQ ID NO: 5 en amont de la séquence codante ou du site de clonage, et la séquence telle que décrite dans SEQ ID NO: 6 en aval de la séquence codante ou du site de clonage.

11. Vecteur baculovirus selon l'une quelconque des revendications 1 à 10, dans lequel la séquence codante code pour un produit thérapeutique.

12. Vecteur baculovirus selon la revendication 11, dans lequel le produit thérapeutique est une protéine, un peptide, un ribozyme, un petit ARN interférent, un microARN ou un ARN antisens.

13. Vecteur baculovirus selon la revendication 12, dans lequel le produit thérapeutique est une protéine ou un peptide impliqué(e) dans le traitement d'un trouble associé au système neural.

14. Vecteur baculovirus selon la revendication 13, dans lequel le trouble associé au système neural est un cancer neuronal, un médulloblastome, un neuroblastome, un gliome, un astrocytome, un trouble neurodégénératif, la maladie d'Alzheimer, la maladie de Parkinson, l'épilepsie ou une lésion résultant d'une exposition à l'alcool.

15. Vecteur baculovirus selon la revendication 13 ou 14, dans lequel le produit thérapeutique est un facteur neurotrophique, un facteur de croissance, une protéine anti-apoptotique, une cytotoxine, une protéine apoptotique, une protéine suppresseur de tumeur, une protéine immunomodulatrice, une oncoprotéine, un anticorps ou une protéine anti-angiogénique.

16. Vecteur baculovirus selon la revendication 15, dans lequel le produit thérapeutique est p53 ou une protéine de la voie p53.

17. Vecteur baculovirus selon la revendication 15, dans lequel le produit thérapeutique est une protéine bactérienne DT-A.

18. Procédé d'expression *in vitro* d'un acide nucléique dans une cellule gliale ou dans une cellule dérivée d'une cellule gliale, comprenant la transfection *in vitro* d'une cellule gliale ou d'une cellule dérivée d'une cellule gliale avec le vecteur baculovirus tel que défini dans l'une quelconque des revendications 1 à 17.

19. Vecteur baculovirus tel que défini dans l'une quelconque des revendications 1 à 17, destiné à être utilisé dans un procédé de traitement d'un trouble associé au système neural chez un sujet, le procédé comprenant l'expression d'un acide nucléique dans une cellule gliale ou dans une cellule dérivée d'une cellule gliale, où le trouble associé aux système neural est un cancer neuronal, un médulloblastome, un neuroblastome, un gliome, un astrocytome, un trouble neurodégénératif, la maladie d'Alzheimer, la maladie de Huntington, la maladie de Parkinson, l'épilepsie, un AVC, une ischémie, un traumatisme de la tête ou de la moelle épinière, la sclérose latérale amyotrophique, un trouble neurogénétique ou une lésion résultant d'une exposition à l'alcool.

20. Vecteur baculovirus destiné à être utilisé dans un procédé de traitement d'un trouble associé au système neural chez un sujet, le vecteur baculovirus comprenant une cassette d'expression, la cassette d'expression comprenant :
un promoteur de la protéine acide fibrillaire gliale (GFAP) en liaison fonctionnelle avec un amplificateur hétérologue ;
une séquence codante en liaison fonctionnelle avec le promoteur de la GFAP ; et
une paire de répétitions terminales inversées virales d'un virus adéno-associé, ladite paire de répétitions terminales inversées virales flanquant au moins le promoteur de la GFAP en liaison fonctionnelle et la séquence codante ;
où l'amplificateur hétérologue augmente l'activité transcriptionnelle gliale-spécifique du promoteur de la GFAP ;
et où le trouble associé au système neural est un cancer neuronal, un médulloblastome, un neuroblastome, un gliome, un astrocytome, un trouble neurodégénératif, la maladie d'Alzheimer, la maladie de Huntington, la maladie de Parkinson, l'épilepsie, un AVC, une ischémie, un traumatisme de la tête ou de la moelle épinière, la sclérose latérale amyotrophique, un trouble neurogénétique ou une lésion résultant d'une exposition à l'alcool.

21. Utilisation d'un vecteur baculovirus pour la préparation d'un médicament destiné au traitement d'un trouble associé au système neural chez un sujet, le vecteur baculovirus comprenant une cassette d'expression, la cassette d'expression comprenant :
un promoteur de la protéine acide fibrillaire gliale (GFAP) en liaison fonctionnelle avec un amplificateur hétérologue ;
une séquence codante en liaison fonctionnelle avec le promoteur spécifique de la GFAP ; et
une paire de répétitions terminales inversées virales d'un virus adéno-associé, ladite paire de répétitions terminales inversées virales flanquant au moins le promoteur de la GFAP en liaison fonctionnelle et la séquence codante ;
où l'amplificateur hétérologue augmente l'activité transcriptionnelle gliale-spécifique du promoteur de la GFAP ;
et où le trouble associé au système neural est un cancer neuronal, un médulloblastome, un neuroblastome, un gliome, un astrocytome, un trouble neurodégénératif, la maladie d'Alzheimer, la maladie de Huntington, la maladie de Parkinson, l'épilepsie, un AVC, une ischémie, un traumatisme de la tête ou de la moelle épinière, la sclérose latérale amyotrophique, un trouble neurogénétique ou une lésion résultant d'une exposition à l'alcool.

22. Vecteur baculovirus selon la revendication 20 ou utilisation selon la revendication 21, où la cassette d'expression est la cassette d'expression telle que définie dans l'une quelconque des revendications 1 à 17.

23. Vecteur baculovirus selon la revendication 20 ou 22, ou utilisation selon la revendication 21 ou 22, où le trouble associé au système neural est un cancer neuronal, un médulloblastome, un neuroblastome, un gliome, un astrocytome, un trouble neurodégénératif, la maladie d'Alzheimer, la maladie de Parkinson, l'épilepsie ou une lésion résultant d'une exposition à l'alcool.

24. Cellule transgénique comprenant le vecteur baculovirus tel que défini dans l'une quelconque des revendications 1 à 17.

25. Animal non humain comprenant le vecteur baculovirus tel que défini selon l'une quelconque des revendications 1 à 17.

26. Composition pharmaceutique comprenant le vecteur baculovirus tel que défini dans l'une quelconque des revendications 1 à 17.
